# EUROPEAN PATENT APPLICATION

(11) **EP 1 666 970 A1**
(43) Date of publication of application: **07.06.2006**
(21) Application number: 04772828.2
(22) Date of filing: 30.08.2004
(51) Int. Cl.: G03F 7/039, C07D 309/12, C07D 311/82, C07D 311/96, C07C 43/23, C07C 69/96, H01L 21/027

(54) **COMPOUND FOR RESIST AND RADIATION-SENSITIVE COMPOSITION**

(30) Priority: 18.09.2003 JP 2003326686; 25.12.2003 JP 2003430459; 15.04.2004 JP 2004119889; 07.05.2004 JP 2004138712
(71) Applicant: MITSUBISHI GAS CHEMICAL COMPANY, INC., Chiyoda-ku, Tokyo 100-8324 (JP)
(72) Inventor: OGURO, Dai, Mitsubishi Gas Chemical Company, Inc., Hiratsuka-shi, Kanagawa 254-0016 (JP); ECHIGO, Masatoshi, Mitsubishi Gas Chemical Co.Inc., Hiratsuka-shi, Kanagawa 254-0016 (JP)
(74) Representative: Gille Hrabal Struck Neidlein Prop Roos
(86) International application number: PCT/JP2004/012879
(87) International publication number: WO 2005/029189

(57) **Abstract**

The compound of the present invention is represented by the following formula 1: 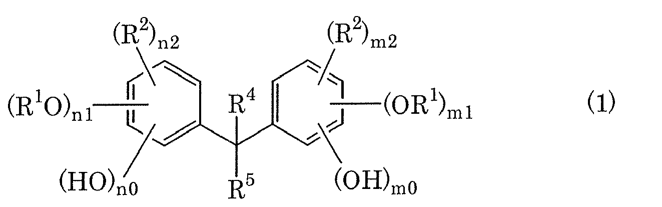 wherein R¹, R², R⁴, R⁵, m0 to m2, and n0 to n2 are as defined in the description. Radiation sensitive compositions containing the compound of the formula 1 as a main component of the solid component are excellent in sensitivity, resolution, heat resistance, etching resistance, and solubility in solvent.

## Description

### Technical Field

The present invention relates to resist compounds useful as acid-amplified non-polymeric resist materials, which have specific chemical structures. The present invention also relates to radiation sensitive compositions comprising such a compound and an acid generator. The resist compounds of the present invention are used as radiation sensitive materials for forming masks, etc. in the production of electronics parts such as LSI and VLSI, which are sensitive to radiations such as ultraviolet rays, far ultraviolet rays, extreme ultraviolet rays (EUV), electron beams and X-rays.

### Background Art

Conventionally known resist materials are generally polymeric materials capable of forming amorphous thin film. For example, a solution of polyhydroxystyrene derivative in a solvent is applied on a substrate to form a thin resist film, which is then irradiated with ultraviolet rays, far ultraviolet rays, electron beams, X-rays, etc., to form line patterns having a line width of about 0.08 µm.

The known polymeric resist materials have molecular weights as large as about 10,000 to 100,000 and broad molecular weight distributions, and their polymer chains are entangled with each other. Therefore, in a finer lithographic process using the polymeric resist materials, the surface of patterns is roughened to make it difficult to control the dimension of patterns, thereby reducing product yields and deteriorating transistor characteristics. Thus, the conventional lithographic techniques using the known polymeric resist materials cannot be applied to the production of fine patterns of 0.06 µm line width or smaller. To produce finer patterns, there have been proposed various resist materials having reduced molecular weights and narrow molecular weight distributions.

Known non-polymeric resist materials include, for example, (1) positive- or negative-type resists derived from fullerene, (2) positive- or negative-type resists derived from calixarene, (3) positive-type resists derived from starburst-type compounds, (4) positive-type resists derived from dendrimers, (5) positive-type resists derived from dendrimers/calixarene, (6) positive-type resists derived from highly branched starburst-type compounds, and (7) positive-type resists derived from ester linkage-containing starburst-type compounds mainly constituted by a trimesic acid structure.

The resist materials (1) are good in etching resistance but not practical in coating properties and sensitivity (JP 7-134413A, JP 9-211862A, JP 10-282649A, JP 11-143074A and JP 11-258796A). The resist materials (2) are excellent in etching resistance, but fail to form satisfactory patterns because of a poor solubility in a developing solution (JP 11-72916A, JP 11-322656A and JP 9-236919A). The resist materials (3) have a low heat resistance and therefore tend to cause the distortion of patterned images during the heat treatment after exposure to light (JP 2000-305270A, JP 2002-99088A and JP 2002-99089A). The resist materials (4) are less practicable because a complicated production process is required and the distortion of patterned images due to their low heat resistance occurs during the heat treatment after exposure to light ("Proceedings of SPIE", vol. 3999 (2000), pp. 1202-1206). The resist materials (5) are less practicable because a complicated production process is required and the raw materials are expensive (JP 2002-49152A and JP 2003-183227A). The resist materials (6) are less practicable because a complicated production process is required, the raw materials are expensive, and metal catalysts unfavorable for the semiconductor production are used. The resist materials (7) are less practicable because the distortion of patterned images due to their low heat resistance is likely to occur during the heat treatment after exposure to light and the adhesion to substrates is poor (JP 2002-328466A).

It has been also disclosed to use a low molecular compound as the additive for light sensitive resin compositions. Proposed compositions include a light sensitive resin composition containing a light sensitive compound having a hydrophobic group or bonding group selected from hydrocarbon groups and heterocyclic groups, and also having a hydrophilic group protected by a protecting group which dissociates by the irradiation of light (JP 2002-363123A), a resist composition containing a low molecular dissolution inhibitor which has two or more triphenylmethane structures at portions other than groups which dissociate by the action of acid (JP 2001-312055A), a resist resin composition containing a light sensitive compound having a fluorene structure (JP 2004-137262A). However, the compositions containing the compound of the invention described below as the main component have not ever been disclosed. Since known resist compositions contain resin, the resist patterns formed therefrom has a large line edge roughness to make the compositions insufficient for practical use. In addition, the compositions containing known resist compounds as the main component involve at least one of the following problems: the film-forming properties are poor because of a high crystallinity; the heat resistance is low to withstand semiconductor process; the compositions are sparingly soluble in safety solvents such as propylene glycol monoethyl ether acetate and ethyl lactate which are acceptable for use in semiconductor factory; and the adhesion to substrate is insufficient. Therefore, known resist compounds are not suitable for single use.

### Disclosure of Invention

An object of the present invention is to provide compounds which are sensitive to radiations such as excimer lasers from KrF, extreme ultraviolet rays (EUV), electron beams and X-rays, and provide radiation sensitive compositions containing such compounds. Another object of the present invention is to provide a simple method of producing non-polymeric radiation sensitive compositions that exhibit a high sensitivity, a high resolution, a high heat resistance, a high etching resistance and a good solubility in solvent.

As a result of extensive study, the inventors have found that the above objects are achieved by the compounds having specific chemical structures and the compositions containing such compounds.

Thus, the present invention provides a radiation sensitive composition comprising from 1 to 80% by weight of a solid component and from 20 to 99% by weight of a solvent, the composition containing a compound B satisfying the following requirements a and b:
(a) having a structure formed by introducing an acid-dissociating functional group into at least one phenolic hydroxyl group of a polyphenol compound A that is produced by a condensation reaction of an aromatic ketone or an aromatic aldehyde each having from 5 to 45 carbon atoms with a compound having from 6 to 15 carbon atoms and from 1 to 3 phenolic hydroxyl groups, and
(b) having a molecular weight of from 300 to 3,000;
and a total content of the compound B and a solubility improver being from 50 to 99.999% by weight of a total weight of the solid component.

The present invention also relates to the compound represented by the following formula 1: 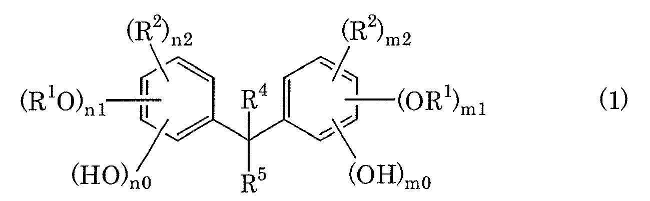 wherein each of R¹ groups is independently an acid-dissociating functional group selected from the group consisting of substituted methyl group, 1-substituted ethyl group, 1-substituted-n-propyl group, 1-branched alkyl group, silyl group, acyl group, 1-substituted alkoxymethyl group, cyclic ether group, and alkoxycarbonyl group;
each of R² group is independently a group selected from the group consisting of halogen atom, alkyl group, cycloalkyl group, aryl group, aralkyl group, alkoxy group, aryloxy group, alkyenyl group, acyl group, alkoxycarbonyl group, alkyloyloxy group, aryloyloxy group, cyano group, and nitro group;
R⁴ is hydrogen atom, C₁₋₆ alkyl group or aryl group, R⁵ is a monovalent C₁₀₋₁₈ group having a biphenyl structure or a naphthalene structure, or -CR⁴R⁵⁻may be a bivalent C₁₀₋₁₈ group having a fluorene structure, a acenaphthene structure, 1-ketoacenaphthene structure or a benzophenone structure when R⁴ and R⁵ are bonded to each other;
m0 and n0 are each an integer of from 0 to 3, m1 and n1 are each an integer of from 0 to 3, and m2 and n2 are each an integer of from 0 to 4, each satisfying the formulae: 1≤ m0 + m1 + m2 ≤ 5, 1 ≤ n0 + n1 + n2 ≤ 5, 1 ≤ m1 + n1 ≤ 6, 1 ≤ m0 + m1≤ 3, and 1 ≤ n0 + n1 ≤ 3; and
two carbon atoms of two benzene rings each being at ortho-position with respect to -CR⁴R⁵- may be bonded to each other via oxygen atom or sulfur atom to form a xanthene structure or a thioxanthene structure represented by the following formula 2: 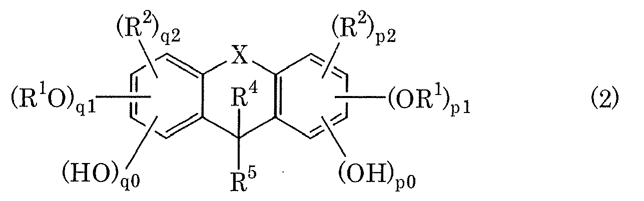 wherein the subscripts R¹, R², R⁴ and R⁵ are the same as defined above; p0 and q0 are each an integer of from 0 to 2, p1 and q1 are each an integer of from 0 to 2, p2 and q2 are each an integer of from 0 to 3, each satisfying 1 ≤ p0 + p1+p2 ≤ 4, 1 ≤q0+q1+q2≤4, 1≤p1+q1≤4, 1≤p0+p1≤2, and 1≤ q0+q1 ≤2; and X is oxygen atom or sulfur atom.

### Best Mode for Carrying Out the Invention

The present invention will be described below in detail.

The radiation sensitive composition of the present invention comprises from 1 to 80% by weight of a solid component and from 20 to 99% by weight of a solvent. The radiation sensitive composition contains a compound B satisfying the following requirements a and b:
(a) having a structure formed by introducing an acid-dissociating functional group into at least one phenolic hydroxyl group of a polyphenol compound A that is produced by a condensation reaction of an aromatic ketone or an aromatic aldehyde each having from 5 to 45 carbon atoms with a compound having from 6 to 15 carbon atoms and from 1 to 3 phenolic hydroxyl groups, and
(b) having a molecular weight of from 300 to 3,000. The total content of the compound B and a solubility improver is from 50 to 99.999% by weight of the total weight of the solid component.

The compound B (resist compound) preferably has a conjugated structure comprising at least two benzene rings and/or a nonbonding electron pair of hetero atom. With such a conjugated structure, the compound B can improve the film-forming properties, enhance the etching resistance, reduce the outgas amount during the exposure to radiations, and increase the sensitivity by its sensitizing effect. The sensitizing effect is attributable to a partial absorption of the energy of radiations such as electron beams by the conjugated structure and an efficient transfer of the absorbed energy to an acid generator.

Examples of the conjugated structures include biphenyl structure, naphthalene structure, fluorene structure, anthracene structure, phenanthrene structure, pyrene structure, benzopyrene structure, acenaphthene structure, acenaphthylene structure, 1-ketoacenaphthene structure, benzophenone structure, xanthene structure, thioxanthene structure, flavone structure, isoflavone structure, indane structure, indene structure, indacene structure, phenalene structure, biphenylene structure, coronene structure, chrysene structure, trinaphthylene structure, hexaphene structure, hexacene structure, rubicene structure, fluoranthene structure, acephenanthrylene structure, perylene structure, picene structure, pentaphene structure, heptaphene structure, heptacene structure, pyranthrene structure, phenacene structure, naphthacene structure, pentacene structure, aceanthrene structure, phenanthrene structure, acephenanthrene structure, azulene structure, triphenylene structure, p-terphenyl structure, m-terphenyl structure, 1,3,5-triphenylbenzene structure, 1,2,3-triphenylbenzene structure, 1,2,4-triphenylbenzene structure, phenylnaphthalene structure, phenylnaphthalene structure, binaphthalene structure, and ovalene structure, with at least one structure selected from biphenyl structure, naphthalene structure, fluorene structure, anthracene structure, pyrene structure, acenaphthene structure, 1-ketoacenaphthene structure, benzophenone structure, xanthene structure, and thioxanthene structure being particularly preferred, because they can be introduced into the compound B using starting compounds of relatively low costs.

The compound B (resist compound) is preferably at least one compound selected from the group consisting of the compounds represented by the following formula 1:

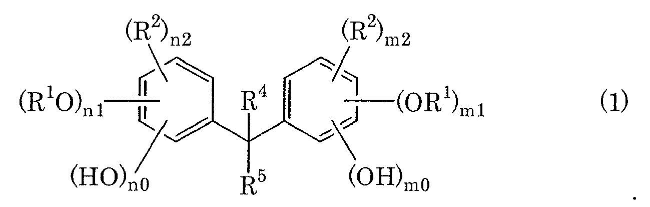

In the formula 1, each of R¹ groups is independently an acid-dissociating functional group selected from the group consisting of substituted methyl group, 1-substituted ethyl group, 1-substituted-n-propyl group, 1-branched alkyl group, silyl group, acyl group, 1-substituted alkoxymethyl group, cyclic ether group, and alkoxycarbonyl group

Examples of the substituted methyl groups include methoxymethyl group, methylthiomethyl group, ethoxymethyl group, ethylthiomethyl group, methoxyethoxymethyl group, benzyloxymethyl group, benzylthiomethyl group, phenacyl group, 4-bromophenacyl group, 4-methoxyphenacyl group, piperonyl group, methoxycarbonylmethyl group, ethoxycarbonylmethyl group, n-propoxycarbonylmethyl group, isopropoxycarbonylmethyl group, n-butoxycarbonylmethyl group, and tert-butoxycarbonylmethyl group.

Examples of the 1-substituted ethyl groups include 1-methoxyethyl group, 1-methylthioethyl group, 1,1-dimethoxyethyl group, 1-ethoxyethyl group, 1-ethylthioethyl group, 1,1-diethoxyethyl group, 1-phenoxyethyl group, 1-phenylthioethyl group, 1,1-diphenoxyethyl group, 1-cyclopentyloxyethyl group, 1-cyclohexyloxyethyl group, 1-phenylethyl group, and 1,1-diphenylethyl group.

Examples of the 1-substituted-n-propyl groups include 1-methoxy-n-propyl group and 1-ethoxy-n-propyl group.

Examples of the 1-branched alkyl groups include isopropyl group, sec-butyl group, tert-butyl group, 1,1-dimethylpropyl group, 1-methylbutyl group, and 1,1-dimethylbutyl group.

Examples of the silyl groups include trimethylsilyl group, ethyldimethylsilyl group, methyldiethylsilyl group, triethylsilyl group, tertbutyldimethylsilyl group, tert-butyldiethylsilyl group, tert-butyldiphenylsilyl group, tri-tert-butylsilyl group, and triphenylsilyl group.

Examples of the acyl groups include acetyl group, phenoxyacetyl group, propionyl group, butyryl group, heptanoyl group, hexanoyl group, valeryl group, pivaloyl group, isovaleryl group, lauroyl group, adamantyl group, benzoyl group, and naphthoyl group.

Examples of the 1-substituted alkoxymethyl groups include 1-cyclopentylmethoxymethyl group, 1-cyclopentylethoxymethyl group, 1-cyclohexylmethoxymethyl group, 1-cyclohexylethoxymethyl group, 1-cyclooctylmethoxymethyl group, and 1-adamantylmethoxymethyl group.

Examples of the cyclic ether groups include tetrahydropyranyl group, tetrahydrofuranyl group, tetrahydrothiopyranyl group, tetrahydrothiofuranyl group, 4-methoxytetrahydropyranyl group, and 4-methoxytetrahydrothiopyranyl group.

Examples of the alkoxycarbonyl groups include methoxycarbonyl group, ethoxycarbonyl group, n-propoxycarbonyl group, isopropoxycarbonyl group, n-butoxycarbonyl group, and tert-butoxycarbonyl group.

Of these acid-dissociating functional groups, preferred are tert-butoxycarbonyl group, tert-butoxycarbonylmethyl group, 1-methoxyethyl group, 1-ethoxyethyl group, 1-cyclohexyloxyethyl group, 1-phenylethyl group, tert-butyl group, trimethylsilyl group, tetrahydropyranyl group, and 1-cyclohexylmethoxymethyl group.

Each of R² groups is independently a group selected from the group consisting of halogen atom, alkyl group, cycloalkyl group, aryl group, aralkyl group, alkoxy group, aryloxy group, alkyenyl group, acyl group, alkoxycarbonyl group, alkyloyloxy group, aryloyloxy group, cyano group, and nitro group.

Examples of the halogen atoms include chlorine atom, bromine atom and iodine atom; examples of the alkyl groups include C₁₋₄ alkyl groups such as methyl group, ethyl group, propyl group, n-propyl group, n-butyl group, isobutyl group, sec-butyl group, and tert-butyl group; examples of the cycloalkyl groups include cyclohexyl group, norbornyl group, and adamantyl group; examples of the aryl groups include phenyl group, tolyl group, xylyl group, and naphthyl group; examples of the aralkyl groups include benzyl group; examples of the alkoxy groups include C₁₋₄ alkoxy groups such as methoxy group, ethoxy group, hydroxyethoxy group, propoxy group, hydroxypropoxy group, isopropoxy group, n-butoxy group, isobutoxy group, sec-butoxy group, and tert-butoxy group; examples of the aryloxy groups include phenoxy group; examples of the alkenyl groups include C₂₋₄ alkenyl groups such as vinyl group, propenyl group, allyl group, and butenyl group; examples of the acyl groups include C₁₋₅ aliphatic acyl groups such as formyl group, acetyl group, propionyl group, butyryl group, valeryl group, isovaleryl group, and pivaloyl group, and aromatic acyl groups such as benzoyl group and toluoyl group; examples of the alkoxycarbonyloxy groups include C₂₋₅ alkoxycarbonyloxy groups such as methoxycarbonyloxy group, ethoxycarbonyloxy group, propoxycarbonyloxy group, isopropoxycarbonyloxy group, n-butoxycarbonyloxy group, isobutoxycarbonyloxy group, secbutoxycarbonyloxy group, and tert-butoxycarbonyloxy group; examples of the alkyloyloxyoxy groups include acetoxy group, propionyloxy group, butyryloxy group, isobutyryloxy group, valeryloxy group, isovaleryloxy group, and pivaloyloxy group; and examples of the aryloyloxyoxy groups include benzoyloxy group.

R² at the o-position of the phenolic hydroxyl group is preferred, because it controls the crystallinity of the compound B to improve the film-forming properties. R² at the o-position of the phenolic hydroxyl group also controls the solubility of the compound B in the alkali developing solution and reduces the degree of introduction of the acid-dissociating functional group into the phenolic hydroxyl groups, thereby improving the solubility in solvent, adhesion to substrate and resist sensitivity. To attain these effects, R² is preferably a bulky and/or electron-donating group, for example, an alkyl group such as methyl group, ethyl group, isopropyl group, t-butyl group, phenyl group, benzyl group, cyclohexyl group, norbornyl group, and adamantyl group; and an alkoxy group such as methoxy group, ethoxy group, isopropoxy group, and phenoxy group. The electron-attracting groups such as halogens, in some cases, increase the solubility of the compound B in alkali developing solutions.

R⁴ is hydrogen atom, C₁₋₆ alkyl group or aryl group, and R⁵ is a monovalent C₁₀₋₁₈ group having a biphenyl structure or a naphthalene structure.

Examples of the C₁₋₆ alkyl group for R⁴ include straight, branched or cyclic alkyl groups such as methyl group, ethyl group, n-propyl group, isopropyl group, n-butyl group, isobutyl group, sec-butyl group, t-butyl group, pentyl group, hexyl group, and cyclohexyl group. The aryl group for R⁴ may be phenyl group.

R⁵ is preferably a monovalent group having a biphenyl structure or a naphthalene structure represented by the following formula: 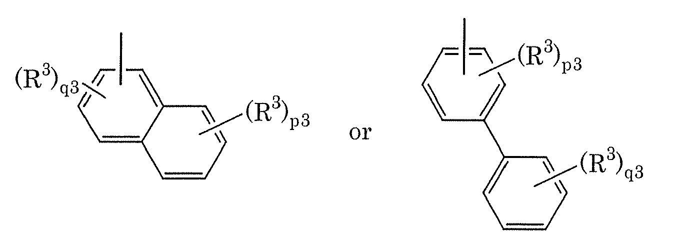 wherein R³ is a hydrogen atom or a C₁₋₆ alkyl group; p3 is an integer of from 0 to 4; q3 is an integer of from 0 to 3; and p3 and q3 satisfy 0 ≤ p3 + q3 ≤7. The C₁₋₆ alkyl group is selected from the alkyl groups mentioned with respect to R⁴.

R⁴ and R⁵ of -CR⁴R⁵- may be bonded to each other to form a bivalent C₁₀₋₁₈ group having a fluorene structure, a acenaphthene structure, 1-ketoacenaphthene structure or a benzophenone structure, preferably a bivalent group represented by the following formula: 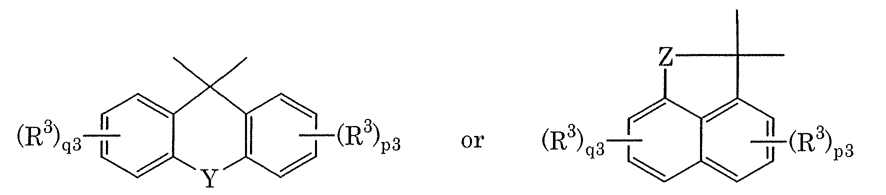 wherein R³, p3 and q3 are the same as defined above, Y is a single bond or a carbonyl group, and Z is a methylene group or a carbonyl group.

Each of m0 and n0 is an integer of from 0 to 3, each of m1 and n1 is an integer of from 0 to 3, and each of m2 and n2 is an integer of from 0 to 4, satisfying 1 ≤ m0 + m1 + m2 ≤ 5, 1 ≤ n0 + n1 + n2 ≤ 5, 1 ≤ m1 + n1 ≤ 6, 1 ≤ m0 + m1 ≤ 3, and 1 ≤ n0 + n1 ≤ 3.

In the formula 1, two carbon atoms each being at o-position of respective two benzene rings with respect to -CR⁴R⁵- may be bonded to each other via an oxygen atom or a sulfur atom to from a xanthene structure or a thioxanthene structure represented by the following formula 2: 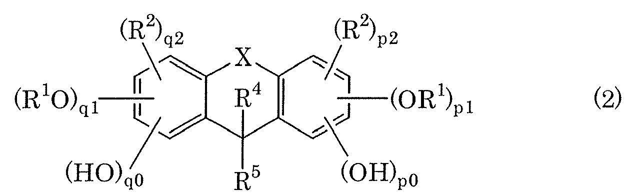 wherein R¹, R², R⁴ and R⁵ are the same as defined above; each of p0 and q0 is an integer of from 0 to 2; each of p1 and q1 is an integer of from 0 to 2; each of p2 and q2 is an integer of from 0 to 3; satisfying 1 ≤ p0 + p1 + p2 ≤ 4, 1 ≤ q0 + q1 + q2≤ 4, 1 ≤ p1+ q1 ≤ 4, 1≤ p0 + p1≤2, and 1≤ q0 + ql ≤ 2; and X is an oxygen atom or a sulfur atom.

The compounds of formula 1, although having a low molecular weight, are excellent in film-forming properties, heat resistance and dry etching resistance, and small in outgas amount. Therefore, resist compositions containing such compounds exhibit a high resolution, a high sensitivity and a small line-edge roughness.

The compound B is preferably at least one compound selected from the compounds represented by the following formulae 3 and 5-12. 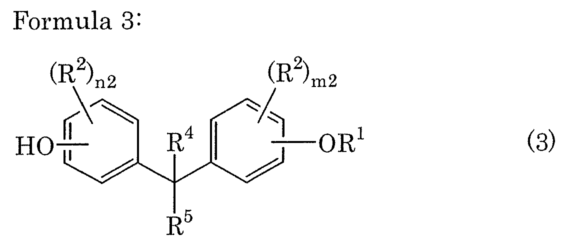 wherein R¹, R², R⁴, R⁵, m2 and n2 are the same as defined above, and two carbon atoms each being at o-position of respective two benzene rings with respect to -CR⁴R⁵- may be bonded to each other via an oxygen atom or a sulfur atom to from a xanthene structure or a thioxanthene structure represented by the following formula 4: 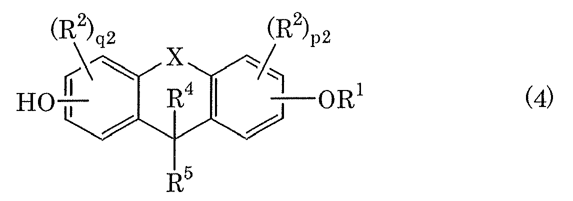 wherein R¹, R², R⁴, R⁵, X, p2 and q2 are the same as defined above.

The compounds of the formula 3 are excellent for practical use because of their good adhesion to substrate, solubility in solvents and heat resistance, and relatively low costs of raw materials. 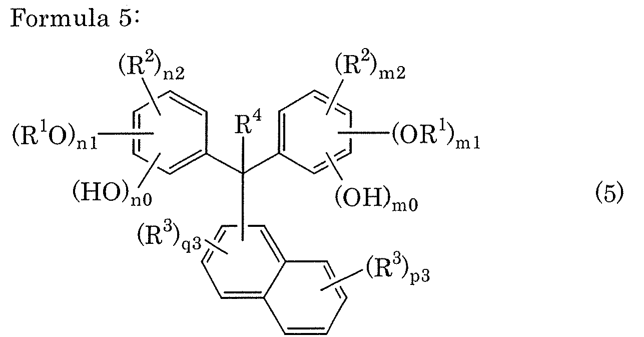 wherein R¹ to R⁴, m0 to m2, n0 to n2, p3, and q3 are the same as defined above. Formula 6: 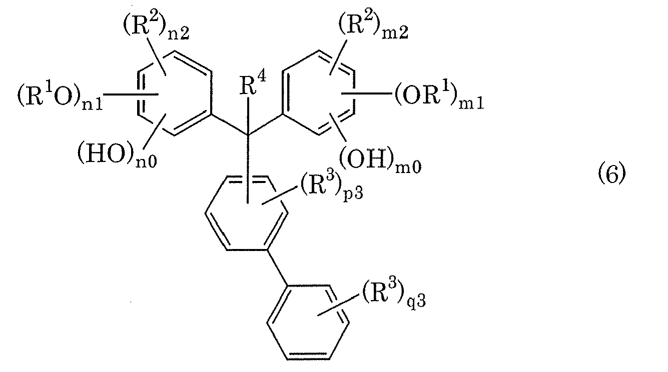 wherein R¹ to R⁴, m0 to m2, n0 to n2, p3, and q3 are the same as defined above. 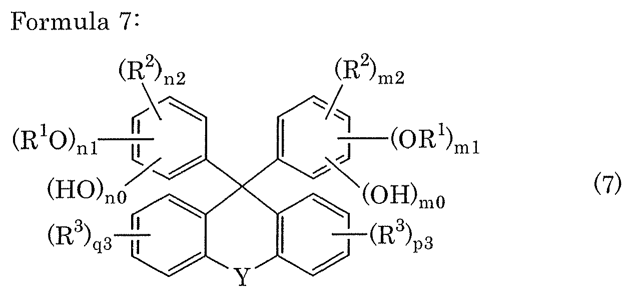 wherein R¹ to R³, Y, m0 to m2, n0 to n2, p3, and q3 are the same as defined above. 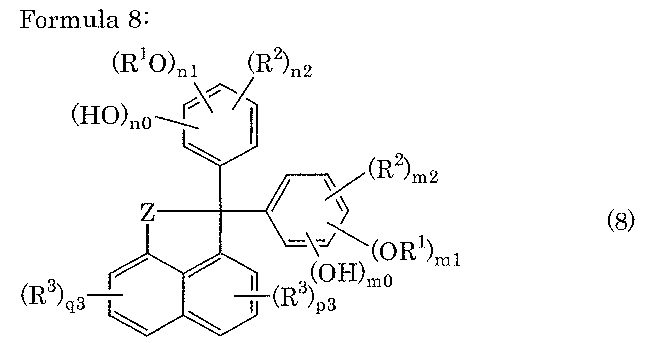 wherein R¹ to R³, Z, m0 to m2, n0 to n2, p3, and q3 are the same as defined above. 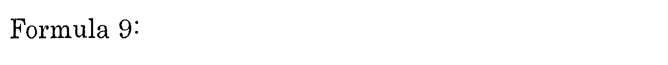 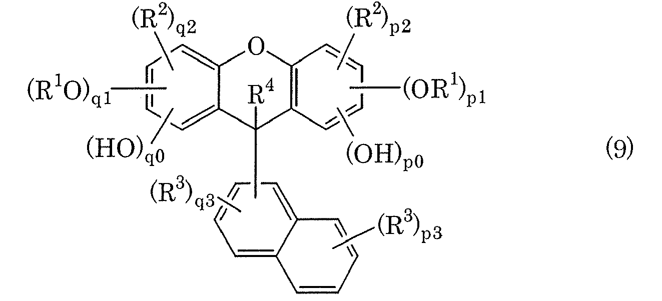 wherein R¹ to R⁴, p0 to p3, and q0 to q3 are the same as defined above. 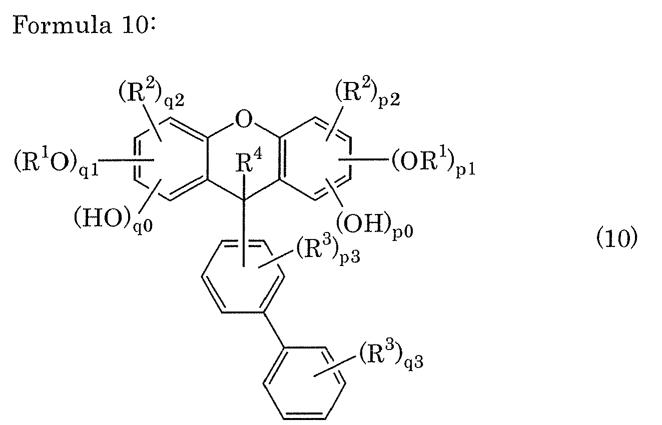 wherein R¹ to R⁴, p0 to p3, and q0 to q3 are the same as defined above. 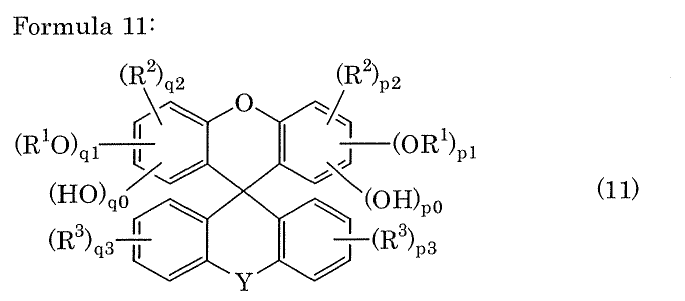 wherein R¹ to R³, Y, p0 to p3, and q0 to q3 are the same as defined above. 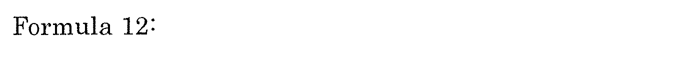 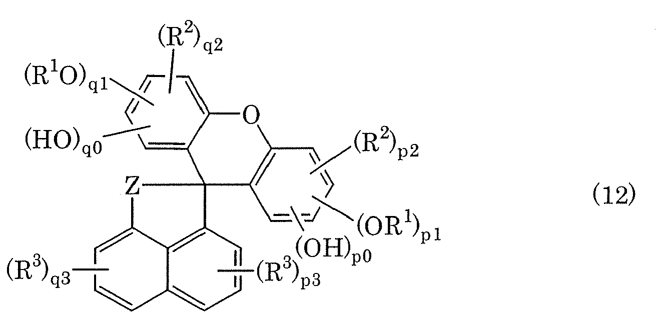 wherein R¹ to R³, Z, p0 to p3, and q0 to q3 are the same as defined above.

In the production of the compound B, a polyphenol compound A is first produced by the condensation of an aromatic ketone or an aromatic aldehyde each having from 5 to 45 carbon atoms with a compound having from 6 to 15 carbon atoms and from 1 to 3 phenolic hydroxyl groups. Then, the acid-dissociating functional group is introduced into at least one phenolic hydroxyl group of the polyphenol compound A, to produce the compound B.

Examples of the aromatic ketone or the aromatic aldehyde each having from 5 to 45 carbon atoms include aromatic ketones such as acetophenone, benzophenone, α-acetonaphthone, β-acetonaphthone, 9-fluorenone, acenaphthenone, benzoquinone, naphthoquinone, anthraquinone, acenaphthenequinone, benzoylbiphenyl, benzoylnaphthalene, acylbiphenyl, acylanthracene, acylphenanthrene, acylphenothiazane, acylpyrene, acylbenzopyrene, acylindacene, acylphenacene, acylacenaphthylene, acylnaphthacene, acylpentacene, acyltriphenylene, acylpyridine, acylimidazole, acylfuran, acylpyrrole, acylovalene, indanone, tetralone, acylthiazole, acridone, flavone, and isoflavone; and aromatic aldehydes such as benzaldehyde, tolylaldehyde, anisaldehyde, 1-naphthoaldehyde, 2-naphthoaldehyde, anthraaldehyde, biphenylaldehyde, formylfluorene, formylbiphenyl, formylanthracene, formylphenanthrene, formylphenothiazane, formylpyrene, formylbenzopyrene, formylindacene, formylphenacene, formylacenaphthylene, formylnaphthacene, formylpentacene, formyltriphenylene, formylpyridine, and formylovalene.

Of the above compounds, particularly preferred are α-acetonaphthone, β-acetonaphthone, 9-fluorenone, acetylanthracene, acetylpyrene, acenaphthenone, acenaphthenequinone, anthraquinone, 1-naphthoaldehyde, and 4-biphenylaldehyde, because of their easy availability with low costs, relatively high reactivity and easy production of the polyphenol compound A.

Examples of the compounds having from 6 to 15 carbon atoms and from 1 to 3 phenolic hydroxyl groups include phenol, (C₁₋₆ alkyl)phenol (for example, cresols such as o-cresol, m-cresol and p-cresol, o-phenylphenol, and 2-cyclohexylphenol), dialkylphenol (for example, 2,3-dimethylphenol, 2,5-dimethylphenol, 2,6-dimethylphenol, and 2,6-di-tert-butylphenol), trialkylphenol, alkoxyphenol (for example, anisoles such as o-methoxyphenol), arylphenol (for example, phenylphenols such as o-phenylphenol and m-phenylphenol), cycloalkylphenol (for example, 2-cyclohexylphenol), halophenols (for example, chlorophenol, dichlorophenol, chlorocresol, bromophenol, dibromophenol), and polyhydric phenols (for example, catechol, alkylcatechol, chlorocatechol, resorcinol, alkylresorcinol, hydroquinone, alkylhydroquinone, chlororesorcinol, chlorohydroquinone, pyrogallol, alkylpyrogallol, phloroglucinol, and 1,2,4-trihydroxyphenol). These compounds may be used alone or in combination of two or more. The purity thereof is, but not specifically limited, usually 95% by weight or more, preferably 99% by weight or more.

Of the above compounds having from 1 to 3 phenolic hydroxyl groups, preferred are phenol, (C₁₋₆ alkyl)phenol, for example, 2-(C₁₋₆ alkyl)phenol (o-cresol, o-phenylphenol, 2-cyclohexylphenol, etc.), catechol, resorcinol, and pyrogallol because of their easy availability. By the use of phenol compounds having at o-positions a bulky group and/or an electron-donating group, the crystallinity of the compound B is controlled to improve the film-forming properties. In addition, the solubility of the compound B in an alkali developing solution is controlled and the degree of introduction of the acid-dissociating functional group into the phenolic hydroxyl groups is reduced, thereby improving the solubility in solvent, adhesion to substrate and resist sensitivity. Examples of the phenol compounds having such effects include 2-(C₁₋₆ alkyl)phenol such as o-cresol, o-phenylphenol, 2-cyclohexylphenol, 2-t-butylphenol, and 2,6-di-t-butylphenol, and 2-alkoxyphenol such as 2-methoxyphenol, 2-isopropoxyphenol, and 2-phenoxyphenol.

The production method of the polyphenol compound A is not particularly limited, and it is produced by the reaction of one mole of the aromatic ketone or the aromatic aldehyde and one mole or excess of the compound having from 1 to 3 phenolic hydroxyl groups such as phenol and o-cresol at 60 to 150°C for about 0.5 to 20 h in the presence of an acid catalyst such as hydrochloric acid and sulfuric acid and a cocatalyst such as thioacetic acid and β-mercaptopropionic acid for preventing the formation of by-products. After the reaction, the reaction production liquid is added with methanol or isopropyl alcohol, stirred for 0.5 to 2 h at 60 to 80°C and then added with an appropriate amount of pure water, to deposit the reaction product. After cooling to room temperature, the deposited reaction product is separated by filtration and dried to obtain the polyphenol compound A. Alternatively, the polyphenol compound A is produced by converting the aromatic ketone or the aromatic aldehyde into a dihalide by a hydrogen halide or a halogen gas, and then, allowing the isolated dihalide to react with the compound having from 1 to 3 phenolic hydroxyl groups.

The compound B is produced, but not limited to, by reacting the polyphenol compound A with a compound for introducing the acid-dissociating functional group such as tert-butoxycarbonyl group and tetrahydropyranyl group at 20 to 60°C under atmospheric pressure for 6 to 24 h in the presence of an amine catalyst such as triethylamine and dimethylaminopyridine or an acid catalyst such as pyridinium tosylate. The reaction product liquid is poured into distilled water to precipitate white solids, which are then washed with distilled water, optionally purified by silica gel column chromatography, and then dried, to obtain the compound B.

Examples of the compound for introducing the acid-dissociating functional group include, but not limited to, acid chlorides, acid anhydrides, dicarbonates, alkyl halides, vinyl alkyl ethers, dihydropyran, each having the acid-dissociating functional group.

The acid-dissociating functional group referred to herein is a functional group that is cleaved in the presence of an acid to generate the phenolic hydroxyl group. To make the formation of resist patterns having still higher sensitivity and resolution possible, it is preferred for the acid-dissociating groups to succeedingly cleave in the presence of acid.

The acid-dissociating functional group is introduced preferably in a proportion of from 10 to 95%, more preferably from 20 to 80% of the total number of the phenolic hydroxyl groups of the polyphenol compound A. Within the above range, the solubility in solvent, adhesion to substrates and sensitivity of the compound B become more good.

The molecular weight of the compound B is from 300 to 3000, preferably from 300 to 1500, and more preferably from 400 to 1000. Within the above range, the resolution is improved while retaining the film-forming properties needed to resists.

The compound B dissolves in propylene glycol monomethyl ether acetate or ethyl lactate preferably in an amount of 5% by weight or more at 23°C. With such a solubility, the safety solvents, which are allowed to use in semiconductor factories, can be used.

The radiation sensitive composition of the present invention comprises from 1 to 80% by weight of the solid compound and from 20 to 99% by weight of the solvent, preferably from 1 to 50% by weight of the solid component and from 50 to 99% by weight of the solvent, and more preferably from 5 to 40% by weight of the solid component and from 60 to 95% by weight of the solvent. The total of the compound B and the solubility improver is from 50 to 99.999% by weight, preferably from 60 to 99% by weight, and more preferably from 80 to 99% by weight, each based on the total weight of the solid component. Within the above range, a high resolution is attained and the line edge roughness becomes small. The content of the solubility improver is preferably from 0 to 80% by weight, more preferably from 20 to 80% by weight, and still more preferably from 30 to 70% by weight, each based on the total weight of the solid component. Within the above range, a high sensitivity and resolution are attained and the line edge roughness becomes small.

Since the radiation sensitive composition of the present invention contains the compound B which meets the requirements a and b, the properties required for resist compositions, such as heat resistance withstanding the semiconductor process, solubility in safety solvents such as propylene glycol monomethyl ether acetate and ethyl lactate, film-forming properties, adhesion to silicon substrate, alkali developability, etching resistance, small amount of outgas during exposure, high resolution and small edge roughness, can be simultaneously achieved.

Examples of the solvent include, but not limited to, ethylene glycol monoalkyl ether acetates such as ethylene glycol monomethyl ether acetate and ethylene glycol monoethyl ether acetate; ethylene glycol monoalkyl ethers such as ethylene glycol monomethyl ether and ethylene glycol monoethyl ether; propylene glycol monoalkyl ether acetates such as propylene glycol monomethyl ether acetate (PGMEA) and propylene glycol monoethyl ether acetate; propylene glycol monoalkyl ethers such as propylene glycol monomethyl ether (PGME) and propylene glycol monoethyl ether; lactic acid esters such as methyl lactate and ethyl lactate (EL); aliphatic carboxylic acid esters such as methyl acetate, ethyl acetate, propyl acetate and butyl acetate; other esters such as methyl 3-methoxypropionate, ethyl 3-methoxypropionate, methyl 3-ethoxypropionate and ethyl 3-ethoxypropionate; aromatic hydrocarbons such as toluene and xylene; ketones such as 2-heptanone, 3-heptanone, 4-heptanone and cyclohexanone; and cyclic ethers such as tetrahydrofuran and dioxane. The solvents may be used singly or in combination of two or more.

In the present invention, the method of generating acid is not particularly restricted as long as acid is surely generated in the system. The use of excimer lasers in place of ultraviolet rays such as g-rays and i-rays enables a finer processing. If high-energy rays such as electron beams, extreme ultraviolet rays, X-rays and ion beams are used, the resist composition can be still more finely processed.

The solid component preferably contains at least one acid generator which generates acid upon irradiation of radiations such as extreme ultraviolet rays (EUV), electron beams and X-rays. The content of the acid generator is preferably from 0.001 to 50% by weight, more preferably from 1 to 40% by weight, and still more preferably from 3 to 20% by weight of the total weight of the solid component. Within the above range, a high sensitivity and a pattern profile with small edge roughness can be attained.

The acid generator is preferably at least one compound selected from the group consisting of the compounds represented by the following formulae 13 to 20, although not limited thereto. 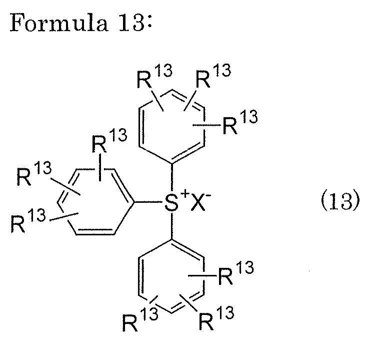 wherein groups R¹³ may be the same or different and are each independently hydrogen, linear, branched or cyclic alkyl group, linear, branched or cyclic alkoxy group, hydroxyl group or halogen; and X⁻ is sulfonic acid ion having alkyl group, aryl group, halogen-substituted alkyl group or halogen-substituted aryl group, or halide ion.

The compound represented by the formula 13 is preferably at least one compound selected from the group consisting of triphenylsulfonium trifluoromethanesulfonate, triphenylsulfonium nonafluoro-n-butanesulfonate, diphenyltolylsulfonium nonafluoro-n-butanesulfonate, triphenylsulfonium perfluoro-n-octanesulfonate, diphenyl-4-methylphenylsulfonium trifluoromethanesulfonate, di-2,4,6-trimethylphenylsulfonium trifluoromethanesulfonate, diphenyl-4-tert-butoxyphenylsulfonium trifluoromethanesulfonate, diphenyl-4-tert-butoxyphenylsulfonium nonafluoro-n-butanesulfonate, diphenyl-4-hydroxyphenylsulfonium trifluoromethanesulfonate, bis(4-fluorophenyl)-4-hydroxyphenylsulfonium trifluoromethanesulfonate, diphenyl-4-hydroxyphenylsulfonium nonafluoro-n-butanesulfonate, bis(4-hydroxyphenyl)phenylsulfonium trifluoromethanesulfonate, tri(4-methoxyphenyl)sulfonium trifluoromethanesulfonate, tri(4-fluorophenyl)sulfonium trifluoromethanesulfonate, triphenylsulfonium p-toluenesulfonate, triphenylsulfonium benzenesulfonate, diphenyl-2,4,6-trimethylphenyl-p-toluenesulfonate, diphenyl-2,4,6-trimethylphenylsulfonium-2-trifluoromethylbenzenesulfonate, diphenyl-2,4,6-trimethylphenylsulfonium-4-trifluoromethylbenzenesulfonate, diphenyl-2,4,6-trimethylphenylsulfonium-2,4-difluorobenzenesulfonate, diphenyl-2,4,6-trimethylphenylsulfonium hexafluorobenzenesulfonate, diphenylnaphthylsulfonium trifluoromethanesulfonate, diphneyl-4-hydroxyphenylsulfonium p-toluenesulfonate, triphenylsulfonium 10-camphorsulfonate and diphenyl-4-hydroxyphenylsulfonium 10-camphorsulfonate. 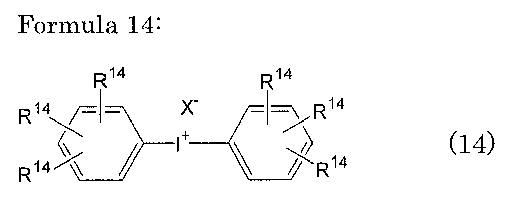 wherein groups R¹⁴ may be the same or different and are each independently hydrogen, linear, branched or cyclic alkyl group, linear, branched or cyclic alkoxy group, hydroxyl group or halogen. X- is the same as defined above.

The compound represented by the formula 14 is preferably at least one compound selected from the group consisting of bis(4-tert-butylphenyl)iodonium trifluoromethanesulfonate, bis(4-tert-butylphenyl)iodonium nonafluoro-n-butanesulfonate, bis(4-tert-butylphenyl)iodonium perfluoro-n-octanesulfonate, bis(4-tert-butylphenyl)iodonium p-toluenesulfonate, bis(4-tert-butylphenyl)iodonium benzenesulfonate, bis(4-tert-but-ylphenyl)iodonium 2-trifluoromethylbenzenesulfonate, bis(4-tert-butylphenyl)iodonium 4-trifluoromethylbenzenesulfonate, bis(4-tert-butylphenyl)iodonium 2,4-difluorobenzenesulfonate, bis(4-tert-butylphenyl)iodonium hexafluorobenzenesulfonate, bis(4-tert-butylphenyl)iodonium 10-camphorsulfonate, diphenyliodonium trifluoromethanesulfonate, diphenyliodonium nonafluoro-n-butanesulfonate, diphenyliodonium perfluoro-n-octanesulfonate, diphenyliodonium p-toluenesulfonate, diphenyliodonium benzenesulfonate, diphenyliodonium 10-camphorsulfonate, diphenyliodonium 2-trifluoromethylbenzenesulfonate, diphenyliodonium 4-trifluoromethylbenzenesulfonate, diphenyliodonium 2,4-difluorobenzenesulfonate, diphenyliodonium hexafluorobenzenesulfonate, di(4-trifluoromethylphenyl)iodonium trifluoromethanesulfonate, di(4-trifluoromethylphenyl)iodonium nonafluoro-n-butanesulfonate, di(4-trifluoromethylphenyl)iodonium perfluoro-n-octanesulfonate, di(4-trifluoromethylphenyl)iodonium p-toluenesulfonate, di(4-trifluoromethylphenyl)iodonium benzenesulfonate and di(4-trifluoromethylphenyl)iodonium 10-camphorsulfonate. 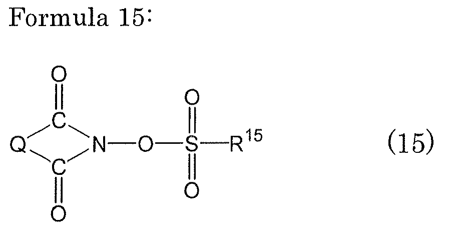 wherein Q is alkylene or arylene, and R¹⁵ is alkyl group, aryl group, halogen-substituted alkyl group or halogen-substituted aryl group.

The compound represented by the formula 15 is preferably at least one compound selected from the group consisting of N-(trifluoromethylsulfonyloxy)succinimide, N-(trifluoromethylsulfonyloxy)phthalimide, N-(trifluoromethylsulfonyloxy)diphenylmaleimide, N-(trifluoromethylsulfonyloxy)bicyclo[2.2.1]hept-5-ene-2,3-dicarboximide, N-(trifluoromethylsulfonyloxy)naphthylimide, N-(10-camphorsulfonyloxy)succinimide, N-(10-camphorsulfonyloxy)phthalimide, N-(10-camphorsulfonyloxy)diphenylmalei.mide, N-(10-camphorsulfonyloxy)bicyclo[2.2.1]hept-5-ene-2,3-dicarboximide, N-(10-camphorsulfonyloxy)naphthylimide, N-(n-octanesulfonyloxy)bicyclo[2.2.1]hept-5-ene-2,3-dicarboximide, N-(n-octanesulfonyloxy)naphthylimide, N-(p-toluenesulfonyloxy)bicyclo[2.2.1]hept-5-ene-2,3-dicarboximide, N-(p-toluenesulfonyloxy)naphthylimide, N-(2-trifluoromthylbenzenesulfonyloxy)bicyclo[2.2.1]hept-5-ene-2,3-dicarboximide, N-(2-trifluoromthylbenzenesulfonyloxy)naphthylimide, N-(4-trifluoromethylbenzenesulfonyloxy)bicyclo[2.2. I]hept.5.ene.2,3.dicarboximide, N-(4-trifluoromethylbenzenesulfonyloxy)naphthylimide, N-(perfluorobenzenesulfonyloxy)bicyclo[2.2.1]hept-5-ene-2,3-dicarboximide, N-(perfluorobenzenesulfonyloxy)naphthylimide, N-(1-naphthalenesulfonyloxy)bicyclo[2.2.1]hept-5-ene-2,3-dicarboximide, N-(1-naphthalenesulfonyloxy)naphthylimide, N-(nonafluoro-n-butanesulfonyloxy)bicyclo[2.2.1]hept-5-ene-2,3-dicarboximide, N-(nonafluoro-n-butanesulfonyloxy)naphthylimide, N-(perfluoro-n-octanesulfonyloxy)bicyclo[2.2.1]hept-5-ene-2,3-dicarboximide and N-(perfluoro-n-octanesulfonyloxy)naphthylimide. 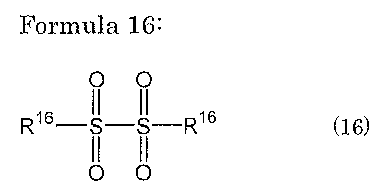 wherein groups R¹⁶ may be the same or different and are each independently linear, branched or cyclic alkyl group which is optionally substituted, aryl group which is optionally substituted, heteroaryl group which is optionally substituted or aralkyl group which is optionally substituted.

The compound represented by the formula 16 is preferably at least one compound selected from the group consisting of diphenyl disulfone, di(4-methylphenyl) disulfone, dinaphthyl disulfone, di(4-tert-butylphenyl) disulfone, di(4-hydroxyphenyl) disulfone, di(3-hydroxynaphthyl) disulfone, di(4-fluorophenyl) disulfone, di(2-fluorophenyl) disulfone and di(4-trifluoroxnethylphenyl) disulfone. 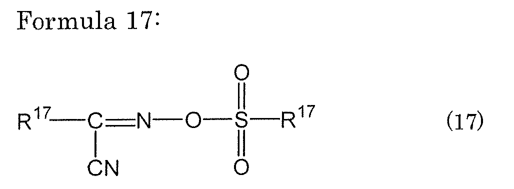 wherein groups R¹⁷ may be the same or different and are each independently linear, branched or cyclic alkyl group which is optionally substituted, aryl group which is optionally substituted, heteroaryl group which is optionally substituted or aralkyl group which is optionally substituted.

The compound represented by the formula 17 is preferably at least one compound selected from the group consisting of α-(methylsulfonyloxyimino)phenylacetonitrile, α-(methylsulfonyloxyimino)-4-methoxyphenylacetonitrile, α-(trifluoromethylsulfonyloxyimino)phenylacetonitrile, α-(trifluoromethylsulfonyloxyimino)-4-methoxyphenylacetonitrile, α-(ethylsulfonyloxyimino)-4-methoxyphenylacetonitrile, α-(propylsulfonyloxyimino)-4-methylphenylacetonitrile and α-(methylsulfonyloxyimino)-4-bromophenylacetonitrile. 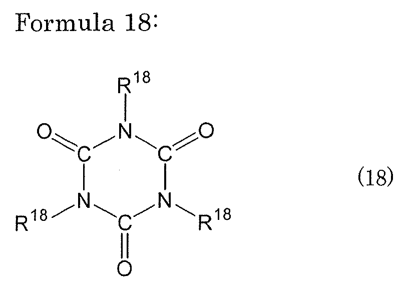 wherein groups R¹⁸ may be the same or different and are each independently halogenated alkyl group having one or more chlorine atoms and one or more bromine atoms. The halogenated alkyl group preferably has from 1 to 5 carbon atoms. 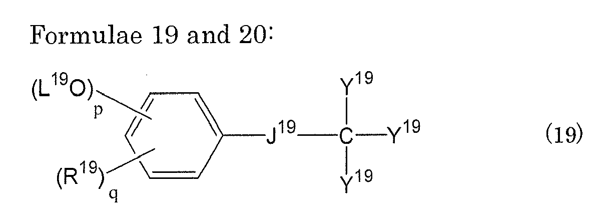 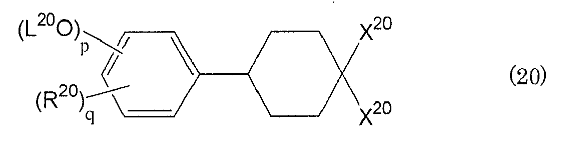 wherein groups R¹⁹ and R²⁰ are each independently C₁ to C₃ alkyl such as methyl group, ethyl group, n-propyl group and isopropyl group, C₃ to C₆ cycloalkyl group such as cyclopentyl group and cyclohexyl group, C₁ to C₃ alkoxy group such as methoxy group, ethoxy group and propoxy group, or aryl group, preferably C₆ to C₁₀ aryl group such as phenyl group, toluyl group and naphthyl group; and groups L¹⁹ and L²⁰ are each independently organic group having a 1,2-naphthoquinonediazido group. Preferred examples of the organic group having a 1,2-naphthoquinonediazido group include 1,2-naphthoquinonediazidosulfonyl groups such as 1,2-naphthoquinonediazido-4-sulfonyl group, 1,2-naphthoquinonediazido-5-sulfonyl group and 1,2-naphthoquinonediazido-6-sulfonyl group, with 1,2-naphthoquinonediazido-4-sulfonyl group and 1,2-naphthoquinonediazido-5-sulfonyl group being more preferred. Subscript p is an integer of 1 to 3 and subscript q is an integer of from 0 to 4, satisfying the formula 1 ≤ p + q≤ 5; J¹⁹ is a single bond, C₁ to C₄ polymethylene group, cycloalkylene group, phenylene group, group represented by the following formula 21: 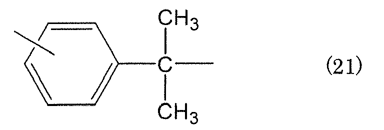 carbonyl group, ester group, amido group or ether group; Y¹⁹ is hydrogen, alkyl group or aryl group; and X¹⁹ and X²⁰ are each independently a group represented by the following formula 22. 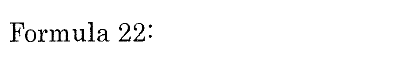 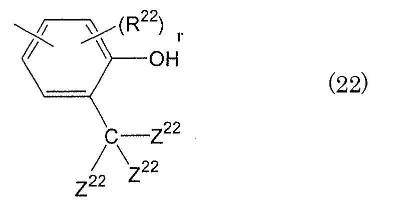 wherein groups Z²² are each independently alkyl group, cycloalkyl group or aryl group; R²² is alkyl group, cycloalkyl group or alkoxy group; and r is an integer of from 0 to 3.

Examples of the other acid generators include bissulfonyldiazomethanes such as bis(p-toluenesulfonyl)diazomethane, bis(2,4-dimethylphenylsulfonyl)diazomethane, bis(tert-butylsulfonyl)diazomethane, bis(n-butylsulfonyl)diazomethane, bis(isobutylsulfonyl)diazomethane, bis(isopropylsulfonyl)diazomethane, bis(n-propylsulfonyl)diazomethane and bis(cyclohexylsulfonyl)diazomethane; and halogen-containing triazine derivatives such as 2-(4-methoxyphenyl)-4,6-(bistrichloromethyl)-1,3,5-triazine, 2-(4-methoxynaphthyl)-4,6-(bistrichloromethyl)-1,3,5-triazine, tris(2,3-dibromopropyl)-1,3,5-triazine and tris(2,3-dibromopropyl)isocyanurate.

The solubility improver is a compound for adequately increasing the dissolving speed of the compound B in the developing operation by increasing the solubility of the compound B in a developing solution such as alkalis, if the solubility is relatively low. It is preferred for the solubility improver to cause no chemical change in the steps of baking of resist film, irradiation of electron beams and development. For example, low-molecular weight phenolic compounds such as bisphenols, tris(hydroxyphenyl)methane and tetrakisphenols are used as the solubility improvers. Examples of the bisphenols include biphenol, bis(4-hydroxyphenyl)propane, bis(4-hydroxyphenyl)methanone, metylenebisphenol, ethylidenebisphenol, cyclohexylidenebisphenol, and phenylethylidenebisphenol. Examples of the trisphenols include tris(4-hydroxyphenyl)methane, tris(4-hydroxyphenyl)ethane, and tris(4-hydroxyphenylbenzene). Examples of the tetrakisphenols include 4,4',4",4"'-(1,2-ethanediylidene)tetrakisphenol, 4,4',4",4"'-(1,2-phenylenedimethylidyne)tetrakisphenol, calix[4]allene, and tetrakis(bicyclohexylidene)phenol. These solubility improvers may be used alone or in combination of two or more. The content of the solubility improver is regulated according to the kind of the compound B used, and preferably from 0 to 80% by weight, more preferably from 20 to 80% by weight, and still more preferably from 30 to 70% by weight of the total weight of the solid component.

The low-molecular weight phenolic compound is preferably represented by the following formula 25: 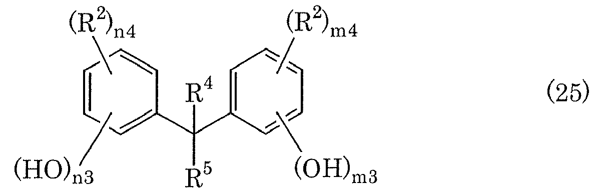 wherein R², R⁴, and R⁵ are as defined above; m3 and n3 are each an integer of from 1 to 3; m4 and n4 are each an integer of from 0 to 4; each satisfying 1 ≤ m3 + m4 ≤ 5 and 1 ≤ n3 + n4 ≤ 5; and two carbon atoms of two benzene rings each being at ortho-position with respect to -CR⁴R⁵- may be bonded to each other via oxygen atom or sulfur atom to form a xanthene structure or a thioxanthene structure represented by the following formula 26: 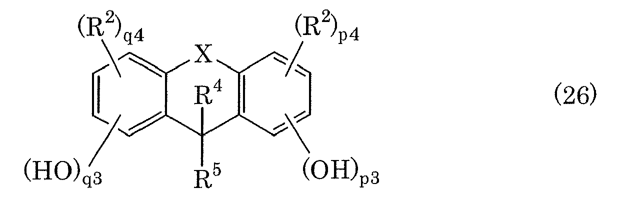 wherein R², R⁴, R⁵, and X are as defined above; p3 and q3 are each an integer of from 1 to 2; and p4 and q4 are each an integer of from 0 to 3.

The radiation sensitive composition of the present invention may further contain various additives such as acid-diffusion controllers, solubility controllers, sensitizers and surfactants.

The acid-diffusion controller has an effect of controlling undesirable chemical reactions in unexposed area by inhibiting the acid generated from the acid generator upon the irradiation of radiation from being diffused throughout the resist film. Using such an acid-diffusion controller, the storage stability and resolution of the radiation sensitive composition can be improved. In addition, the change of line width of resist patterns due to the variation of time delay before and after irradiation of electron beams, etc. can be prevented thereby to make the process stability extremely excellent. Examples thereof include nitrogen-containing basic compounds and basic compounds degradable by the irradiation of electron beams such as basic sulfonium compounds and basic iodonium compounds. The acid-diffusion controllers may be used singly or in combination of two or more.

The content of the acid-diffusion controller is preferably from 0 to 10% by weight, more preferably from 0.001 to 5% by weight and still more preferably from 0.001 to 3% by weight of the total weight of the solid component. If less than 0.001% by weight, the resolution, pattern profiles and dimension accuracy may be deteriorated in some processing conditions. In addition, the proper pattern profile may be lost, if the time delay from the irradiation of electron beams until the post-irradiation heating is prolonged. If exceeding 10% by weight, the sensitivity of resist composition and the developability of unexposed area may be deteriorated.

The solubility controller is a compound for adequately reducing the dissolving speed of the compound B into a developing solution such as alkalis by lowering the solubility, if the solubility is relatively high. It is preferred for the solubility controller to cause no chemical change in the steps of baking of resist film, irradiation of radiation and development.

Examples of the solubility controller include aromatic hydrocarbons such as naphthalene, phenanthrene, anthracene and acenaphthene; ketones such as acetophenone, benzophenone and phenyl naphthyl ketone; and sulfones such as methyl phenyl sulfone, diphenyl sulfone and dinaphthyl sulfone. The solubility controllers may be used singly or in combination of two or more. The content of the solubility controller varies depending upon the kind of the compound B used, and is preferably from 0 to 50% by weight, more preferably from 0 to 50% by weight, more preferably from 0.1 to 20% by weight, and still more preferably from 1 to 10% by weight of the total weight of the solid component.

The sensitizer is a compound for increasing the generation of acid by absorbing the energy of irradiated radiation and transferring it to the acid generator, thereby enhancing the apparent sensitivity of the resist. Examples of the sensitizer include, but not limited to, benzophenones, biacetyls, pyrenes, phenothiazines, and fluorenes. The sensitizers may be used singly or in combination of two or more. The content of the sensitizer is preferably from 0.1 to 20% by weight, and still more preferably from 1 to 10% by weight of the total weight of the solid component.

The surfactant is a compound for improving the coating property and striation of the radiation sensitive composition and the developability of the resist, etc. As the surfactant, there may be used any of anionic, cationic, nonionic and ampholytic surfactants, with nonionic surfactants being preferred because of their good affinity to solvents used for the radiation-sensitive composition. Examples of the nonionic surfactants include, but not particularly limited to, polyoxyethylene higher alkyl ethers, polyoxyethylene higher alkyl phenyl ethers, and higher fatty acid diesters of polyethylene glycol, which are commercially available under the following tradenames: "EFTOP" of Jemco Inc.; "MEGAFACE" of Dai-Nippon Ink & Chemicals, Incorporated; "FLUORAD" of Sumitomo 3M Ltd.; "ASAHIGUARD" and "SURFLON" of Asahi Glass Co., Ltd.; "PEPOL" of Toho Chemical Industry Co., Ltd.; "KP" of Shin-Etsu Chemical Co., Ltd.; and "POLYFLOW" of Kyoeisha Chemical Co., Ltd.

The content of the surfactant is preferably from 0 to 2% by weight, more preferably from 0.0001 to 1% by weight, and still more preferably from 0.001 to 0.1% by weight of the total weight of the solid component. In addition, dyes or pigments may be blended in the radiation sensitive composition to visualize latent images of exposed portions, thereby reducing adverse influence of halation during the exposing operation. Further, adhesive assistants may be blended in the radiation sensitive composition to improve the adhesion to substrates.

In addition to the additives mentioned above, the radiation sensitive composition may further contain a resin which is water-insoluble but soluble in aqueous alkali solutions or a resin which is water-insoluble but becomes soluble in aqueous alkali solutions by the action of acid to acquire the developability by aqueous alkali solutions. Examples of such resins include phenol resins to which the acid-dissociating functional group may be introduced; novolak resins to which the acid-dissociating functional group may be introduced; hydrogenated novolak resins to which the acid-dissociating functional group may be introduced; o-polyhydroxystyrene, m-polyhydroxystyrene, p-polyhydroxystyrene and their copolymers, to each of which the acid-dissociating functional group may be introduced; alkyl-substituted polyhydroxystyrene to which the acid-dissociating functional group may be introduced; polyhydroxystyrene to which the acid-dissociating functional group may be introduced; partially o-alkylated polyhydroxystyrene to which the acid-dissociating functional group may be introduced; styrene-hydroxystyrene copolymers to which the acid-dissociating functional group may be introduced; α-methylstyrene-hydroxystyrene copolymers to which the acid-dissociating functional group may be introduced; polyalkyl methacrylate to which the acid-dissociating functional group may be introduced; polyolefin; polyester; polyamide; polyurea; and polyurethane. Since the line edge roughness increases with increasing addition amount of the resin, a good pattern profile tends to be obtained when the use of resin is rather avoided than be used.

The radiation sensitive composition of the invention is produced by mixing the compound B, solvent, solubility improver and optional additives such as an acid generator under stirring. The amount of each optional additive, if used, is selected from the range mentioned above so that the total content of the components in the solid component adds up to 100% by weight. The stirring method, the mixing method and the order of mixing are not critical, and one of skilled in the art could easily select them.

In the formation of a resist pattern using the radiation sensitive composition of the invention, the radiation sensitive composition is first applied on a substrate such as a silicon wafer, a gallium-arsenic wafer and an aluminum-coated wafer by a coating method such as rotational coating, cast coating and roll coating to form a resist film.

The substrate may be treated with a surface treating agent, if necessary. Examples of the surface treating agents include a silane coupling agent such as hexamethylenedisilazane (for example, silane coupling agent polymerizable under hydrolysis), an anchor coating agent or primer (for example, polyvinyl acetal, acrylic resins, vinyl acetate resins, epoxy resins and urethane resins), and a coating agent comprising a mixture of the primer and inorganic fine particles.

The thickness of the resist film is preferably from 0.01 to 10 µm, more preferably from 0.05 to 1 µm, and still more preferably from 0.08 to 0.5 µm, although not limited thereto.

If necessary, a resist protecting film may be formed after forming the resist film so as to prevent suspended amines, etc. in air from entering into the resist film. The acid generated by the exposure to radiation is deactivated when allowed to react with a compound which is reactive to acid such as amines suspended in air as impurities. With such a resist protecting film, the deterioration of resist image and the reduction of sensitivity due to such a deactivation can be prevented. The resist protecting film is preferably formed from a water-soluble, acidic polymer such as polyacrylic acid and polyvinyl sulfonate.

The resist film is then exposed to radiation such as ultraviolet rays, extreme ultraviolet rays (EUV) and electron beams in desired patterns. The exposed compound B changes to a compound soluble in an alkali developing solution, because its acid-dissociating functional group is cleaved to form a phenolic hydroxyl group. The conditions for exposure may be selected depending on the formulation, etc. of the radiation-sensitive composition. In the present invention, it is preferred to conduct a heat treatment after the irradiation of radiation to stably form highly accurate fine patterns by the exposure. The heating temperature is preferably from 20 to 250°C and more preferably from 40 to 150°C, although it depends upon the formulation, etc. of the radiation sensitive composition.

Then, the exposed resist film is developed with an alkali developing solution to form desired resist patterns. As the alkali developing solution, there may be used an aqueous alkaline solution dissolving, for example, at least one alkaline compound selected from mono-, di- or trialkylamines, mono-, di- or trialkanolamines, heterocyclic amines, tetramethylammonium hydroxide (TMAH) and choline in a concentration of preferably from 1 to 10% by mass and more preferably from 1 to 5% by mass.

Further, the alkali developing solution may contain an appropriate amount of an alcohol such as methanol, ethanol and isopropyl alcohol, or a surfactant mentioned above, with the addition of isopropyl alcohol in 10 to 30% by mass being particularly preferred. After developing with such an aqueous alkaline solution, the developed patterns are usually washed with water.

After forming resist patterns, the substrate is subjected to etching to obtain a patterned wiring board. The etching may be performed by known methods such as dry-etching using a plasma gas and wet-etching using an alkali solution, a copper (II) chloride solution, an iron (III) chloride solution, etc.

After forming resist patterns, the substrate may be plated, for example, by copper plating, solder plating, nickel plating or gold plating.

The remaining resist patterns may be stripped off by an organic solvent or an alkaline aqueous solution stronger than the aqueous alkali solution used for the development. Examples of the organic solvent include PGMEA, PGME and EL. Examples of the strong alkaline aqueous solution include 1 to 20% by mass aqueous sodium hydroxide solution and 1 to 20% by mass aqueous potassium hydroxide solution. The stripping of the resist patterns may be performed by dipping method, spray method, etc. The wiring board having the resist patterns thereon may be a multi-layer wiring board and may be formed with small through-holes.

The wiring board may be produced by a lift off method in which a metal is vacuum-deposited after the formation of resist patterns and then the remaining resist patterns are removed by dissolution into a solution.

The present invention will be described in more detail with reference to the following examples. However, it should be noted that the following examples are only illustrative and do not limit the scope of the present invention thereto.

The compounds, radiation sensitive compositions and resist patterns were evaluated by the following methods.

### I Evaluation of Compounds and Radiation Sensitive Compositions

### (1) Solubility of Compound in Safety Solvent

Each compound was tested for its solubility in safety solvent (PGMEA or EL) at 23°C. The solubility was rated as "A" when dissolved in an amount of 5% by weight or more, "B" when dissolved in an amount of 0.1% by weight or more but less than 5% by weight, and "C" when not dissolved in an amount of 0.1% by weight or more.

### (2) Film-Forming Properties of Radiation Sensitive Compositions

A 10% by weight solution of each compound in PGMEA was spin-coated on a silicon wafer by using a spin coater, to form a resist film of about 0.2 µm thick. After heating on a hot plate for 3 min at about 110°C, the state of the resist film was observed. The results were rated as "C" when the resist film was whitened or its surface was roughened, "B" when partially whitened or partially roughened, and "A" when the surface appeared good.

### (3) Inhibition of Dissolution in Alkali Developing Solution

The resist film formed in the evaluation 2 was immersed in a developing solution (2.38% aqueous solution of TMAH) for 3 min. The results were rated as "A" when no change was visually noticed in the state of film, and "C" when the surface was roughened or a part of the film was lost because of dissolution.

### (4) Adhesion to Silicon Substrate

The adhesion was rated as "A" when the resist film formed in the evaluation 2 did not peel off the silicon wafer, "B" when did not peel off when a surface treating agent (silane coupling agent) was used, and "C" when peeled off even when a surface treating agent was used.

### (5) Alkali Developability

A polyphenol compound A corresponding to each compound before introducing the acid-dissociating functional group was examined for its developability with an alkali developing solution.

A 10% by weight solution of each polyphenol compound A in PGMEA was spin-coated using a spin coater, to form a resist film of about 0.2 µm thick. After heating on a hot plate for 3 min at 110°C, the resist film was immersed in a 2.38% aqueous solution of TMAH for 10 s. The results were rated as "A" when the resist film dissolved completely and disappeared, and "C" when the resist film remained not dissolved even in a slight amount.

### II Evaluation of Resist Pattern

### (1) Formation of Resist Film

Each formulation shown in Table 5 was filtered through a 0.2-µm Teflon (registered trademark) filter to prepare a radiation sensitive composition. Each radiation sensitive composition was coated on a silicon wafer using a spin coater, and then dried on a hot plate for 60 s at 110°C, to form a resist film of about 0.2 µm thick.

### (2) Patterning of Resist Film

The exposure to radiation of the resist film was conducted using an electron beam lithography system (accelerating voltage: 50 keV). After the exposure, the resist film was heated for 60 s at a post-exposure baking temperature (PEB) shown in Table 6. Then, the resist film was immersed in an 2.38% aqueous solution of TMAH for 30 s, rinsed with distilled water for 30 s, and then dried. The resultant single line or a line-and-space pattern was observed under a scanning electron microscope.

### (3) Evaluation of Sensitivity and Resolution

A limiting resolution on the single line or the line-and-space pattern was employed as the resolution. The sensitivity was expressed by a minimum exposure which provided the limiting resolution.

### EXAMPLE 1: Synthesis of Compound 5-1

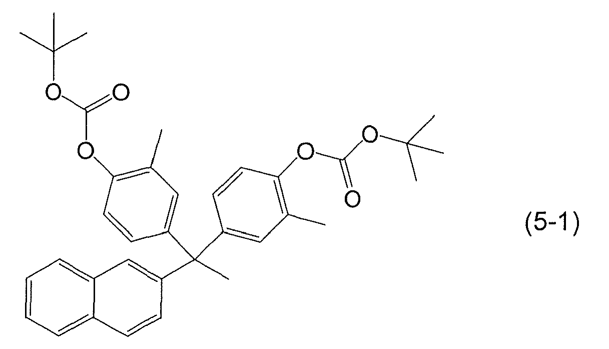

### (1) Synthesis of 1-(2-naphthyl)-1,1-bis(3-methyl-4-hydroxyphenyl)ethane

Into a solution prepared by heating a mixture of 43.2 g (0.4 mol) of o-cresol (reagent available from Kanto Chemical Co., Inc.) and 17.1 g (0.1 mol) of β-acetonaphthone (reagent available from Kanto Chemical Co., Inc.) to about 30°C, were added 0.1 ml of sulfuric acid, 0.8 ml of 3-mercaptopropionic acid and 10 ml of toluene. The reaction was allowed to proceed under stirring. After confirming that the conversion reached 100% by gas chromatography, 100 ml of toluene was added. After cooling, the precipitated solid was separated by vacuum filtration, washed with warm water of 60°C under stirring and purified by silica gel column chromatography, to obtain 24 g of the titled compound.

### (2) Synthesis of Compound 5-1

Into a solution prepared by mixing 1.84 g (5 mmol) of 1-(2-naphthyl)-1,1-bis(3-methyl-4-hydroxyphenyl)ethane synthesized above with 5 ml of anhydrous acetone and 1.2 mg of dimethylaminopyridine (reagent available from Kanto Chemical Co., Inc.), 2.62 g (12 mmol) of di-tert-butyl dicarbonate (reagent available from Kanto Chemical Co., Inc.) was added dropwise over 10 min. The resultant mixture was stirred for 24 h at 40°C. The reaction liquid was poured into an excessive amount of water to precipitate solid matters. The obtained white powder was washed with distilled water three times, filtered under suction, and dried under reduced pressure, to obtain the titled compound. The structure of the compound was determined by elemental analysis and ¹H-NMR measurement. The results of the analysis are shown in Tables 1 and 2.

### EXAMPLE 2: Synthesis of Compound 5-2

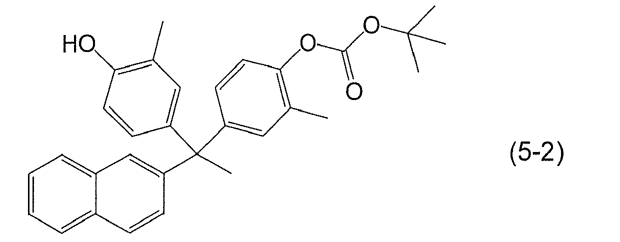

Into a solution prepared by mixing 1.84 g (5 mmol) of 1-(2-naphthyl)-1,1-bis(3-methyl-4-hydroxyphenyl)ethane synthesized in Example 1 with 5 ml of anhydrous acetone and 1.2 mg of dimethylaminopyridine, 1.32 g (6 mmol) of di-tert-butyl dicarbonate was added dropwise over 10 min. The resultant mixture was stirred for 24 h at 40°C. The reaction liquid was purified by a silica gel column chromatography (eluent: ethyl acetate/hexane = 1/3) to obtain the titled compound. The structure of the compound was determined by elemental analysis and ¹H-NMR measurement. The results of the analysis are shown in Tables 1 and 2.

### EXAMPLE 3: Synthesis of Compound 5-3

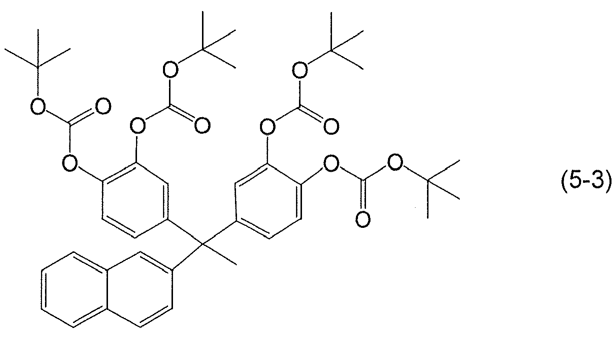

### (1) Synthesis of biscatechol compound

In the same manner as in Example 1(1) except for using 44.0 g (0.4 mol) of catechol (reagent available from Kanto Chemical Co., Inc.) in place of 43.2 g (0.4 mol) of o-cresol, 1-(2-naphthyl)-1,1-bis(3,4-dihydroxyphenyl)ethane was synthesized.

### (2) Synthesis of Compound 5-3

Into a solution prepared by mixing 1.84 g (5 mmol) of 1-(2-naphthyl)-1,1-bis(3,4-dihydroxyphenyl)ethane synthesized above with 5 ml of anhydrous acetone and 1.2 mg of dimethylaminopyridine, 5.28 g (24 mmol) of di-tert-butyl dicarbonate was added dropwise over 10 min. The resultant mixture was stirred for 24 h at 40°C. The reaction liquid was poured into an excessive amount of water to precipitate solid matters. The obtained white powder was washed with distilled water three times, filtered under suction, and dried under reduced pressure, to obtain the titled compound. The structure of the compound was determined by elemental analysis and ¹H-NMR measurement. The results of the analysis are shown in Tables 1 and 2.

### EXAMPLE 4: Synthesis of Compound 5-4

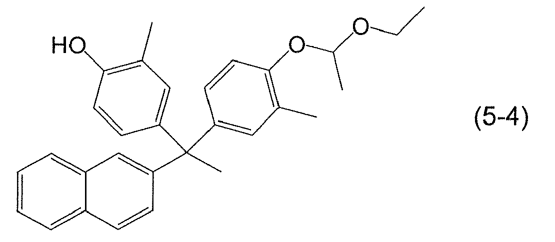

A solution prepared by mixing 1.84 g (5 mmol) of 1-(2-naphthyl)-1,1-bis(3-methyl-4-hydroxyphenyl)ethane synthesized in Example 1 with 5 ml of anhydrous acetone, 0.073 g (0.29 mmol) of pyridinium p-toluenesulfonate (reagent available from Kanto Chemical Co., Inc.) and 0.43 g (6 mmol) of ethyl vinyl ether (reagent available from Kanto Chemical Co., Inc.) was stirred for 24 h at room temperature. The reaction liquid was purified by a silica gel column chromatography (eluent: ethyl acetate/hexane = 1/3) to obtain the titled compound. The structure of the compound was determined by elemental analysis and ¹H-NMR measurement. The results of the analysis are shown in Tables 1 and 2.

### EXAMPLE 5: Synthesis of Compound 5-5

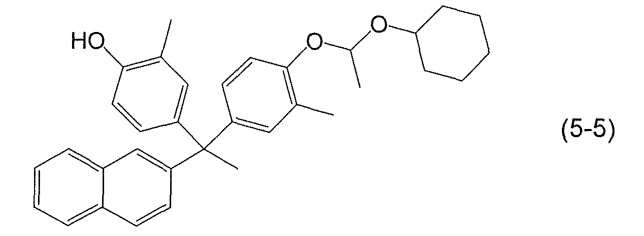

A solution prepared by mixing 1.84 g (5 mmol) of 1-(2-naphthyl)-1,1-bis(3-methyl-4-hydroxyphenyl)ethane synthesized in Example 1 with 5 ml of anhydrous acetone, 0.073 g (0.29 mmol) of pyridinium p-toluenesulfonate and 0.76 g (6 mmol) of cyclohexyl vinyl ether was stirred for 24 h at room temperature. The reaction liquid was purified by a silica gel column chromatography (eluent: ethyl acetate/hexane = 1/3) to obtain the titled compound. The structure of the compound was determined by elemental analysis and ¹H-NMR measurement. The results of the analysis are shown in Tables 1 and 2.

### EXAMPLE 6: Synthesis of Compound 5-6

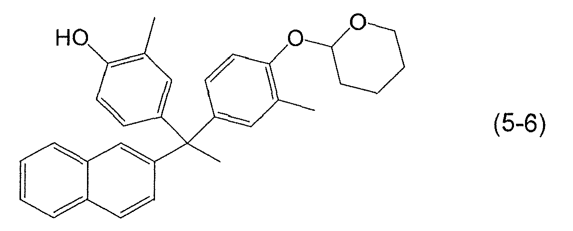

A solution prepared by mixing 1.84 g (5 mmol) of 1-(2-naphthyl)-1,1-bis(3-methyl-4-hydroxyphenyl)ethane synthesized in Example 1 with 5 ml of anhydrous acetone, 0.073 g (0.29 mmol) of pyridinium p-toluenesulfonate and 0.50 g (6 mmol) of dihydropyran (reagent available from Kanto Chemical Co., Inc.) was stirred for 24 h at room temperature. The reaction liquid was purified by a silica gel column chromatography (eluent: ethyl acetate/hexane = 1/3) to obtain the titled compound. The structure of the compound was determined by elemental analysis and ¹H-NMR measurement. The results of the analysis are shown in Tables 1 and 2.

### EXAMPLE 7: Synthesis of Compound 5-7

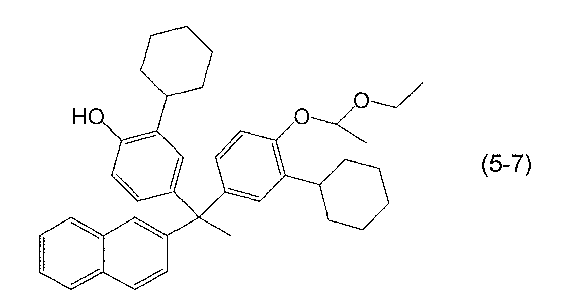

### (1) Synthesis of 1-(2-naphthyl)-1,1-bis(3-cyclohexyl-4-hydroxyphenyl)ethane

In the same manner as in Example 1(1) except for using 53.0 g (0.3 mol) of 2-cyclohexylphenol (reagent available from Honshu Chemical Industry Co., Ltd.) in place of 43.2 g (0.4 mol) of o-cresol, the titled compound was synthesized.

### (2) Synthesis of Compound 5-7

A solution prepared by mixing 2.52 g (5 mmol) of 1-(2-naphthyl)-1,1-bis(3-cyclohexyl-4-hydroxyphenyl)ethane synthesized above with 5 ml of anhydrous acetone, 0.073 g (0.29 mmol) of pyridinium p-toluenesulfonate and 0.43 g (6 mmol) of ethyl vinyl ether was stirred for 24 h at room temperature. The reaction liquid was purified by a silica gel column chromatography (eluent: ethyl acetate/hexane = 1/4) to obtain the titled compound. The structure of the compound was determined by elemental analysis and ¹H-NMR measurement. The results of the analysis are shown in Tables 1 and 2.

### EXAMPLE 8: Synthesis of Compound 5-8

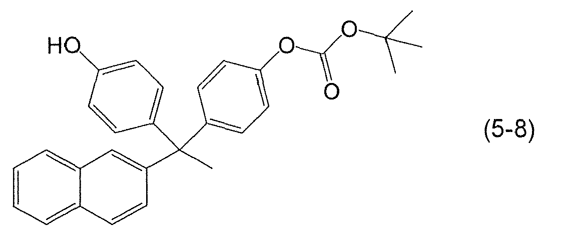

### (1) Synthesis of 1-(2-naphthyl)-1,1-bis(4-hydroxyphenyl)ethane

In the same manner as in Example 1(1) except for using 37.4 g (0.4 mol) of phenol in place of 43.2 g (0.4 mol) of o-cresol, bisphenolacetonaphthone was synthesized.

### (2) Synthesis of Compound 5-8

In the same manner as in Example 1(2) except for using 1.70 g (5 mmol) of 1-(2-naphthyl)- 1,1-bis(4-hydroxyphenyl)ethane in place of 1.84 g (5 mmol) of 1-(2-naphthyl)-1,1-bis(3-methyl-4-hydroxyphenyl)ethane, the titled compound was synthesized. The structure of the compound was determined by elemental analysis and ¹H-NMR measurement. The results of the analysis are shown in Tables 1 and 2.

### EXAMPLE 9: Synthesis of Compound 5-9

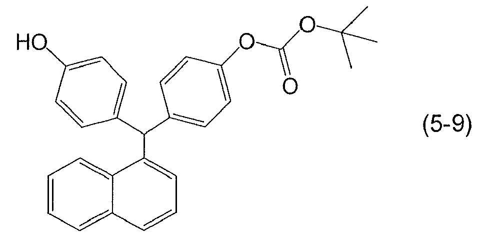

### (1) Synthesis of 1-(1-naphthyl)-1,1-bis(4-hydroxyphenyl)methane

In the same manner as in Example 1(1) except for using 15.6 g (0.1 mol) of α-naphthoaldehyde (reagent available from Kanto Chemical Co., Inc.) in place of 17.1 g (0.1 mol) of β-acetonaphthone, 1-(1-naphthyl)-1,1-bis(4-hydroxyphenyl)methane was synthesized.

### (2) Synthesis of Compound 5-9

In the same manner as in Example 1(2) except for using 1.63 g (5 mmol) of 1-(1-naphthyl)-1,1-bis(4-hydroxyphenyl)methane in place of 1.84 g (5 mmol) of 1-(2-naphthyl)-1,1-bis(3-methyl-4-hydroxyphenyl)ethane, the titled compound was synthesized. The structure of the compound was determined by elemental analysis and ¹H-NMR measurement. The results of the analysis are shown in Tables 1 and 2.

### EXAMPLE 10: Synthesis of Compound 6-1

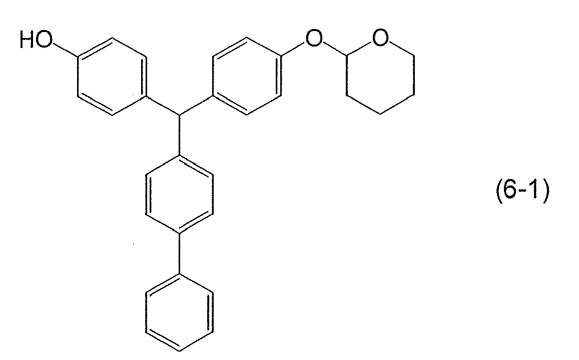

### (1) Synthesis of 1-(4'-biphenyl)-1,1-bis(4-hydroxyphenyl)methane

In the same manner as in Example 1(1) except for using 18.2 g (0.1 mol) of 4'-biphenylaldehyde (available from Mitsubishi Gas Chemical Company, Inc.) in place of 17.1 g (0.1 mol) of β-acetonaphthone, biphenylaldehyde was synthesized.

### (2) Synthesis of Compound 6-1

In the same manner as in Example 6 except for using 1.76 g (5 mmol) of 1-(4'-biphenyl)-1,1-bis(4-hydroxyphenyl.)m.ethane synthesized above in place of 1.84 g (5 mmol) of 1-(2-naphthyl)-1,1-bis(3-methyl-4-hydroxyphenyl)ethane, the titled compound was synthesized. The structure of the compound was determined by elemental analysis and ¹H-NMR measurement. The results of the analysis are shown in Tables 1 and 2.

### EXAMPLE 11: Synthesis of Compound 7-1

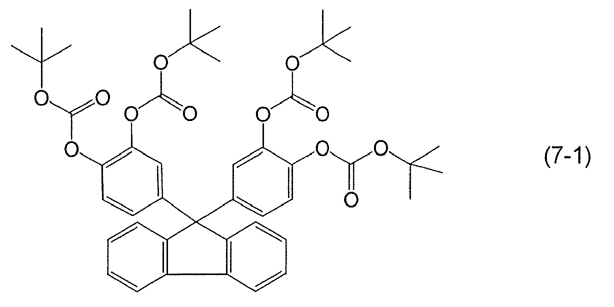

Into a solution prepared by mixing 1.91 g (5 mmol) of biscatecholfluorene (available form Osaka Gas Chemicals Co., Ltd.) with 5 ml of dimethylacetamide (DMAc), 4.80 g (22 mmol) of di-tert-butyl dicarbonate and 2.4 g of triethylamine were slowly added. The resultant mixture was stirred for 7 h at 60°C. The reaction liquid was poured into an excessive amount of water and the reprecipitation was repeated. The obtained white powder was dried under reduced pressure to obtain the titled compound. The structure of the compound was determined by elemental analysis and ¹H-NMR measurement. The results of the analysis are shown in Tables 1 and 2.

### EXAMPLE 12: Synthesis of Compound 7-2

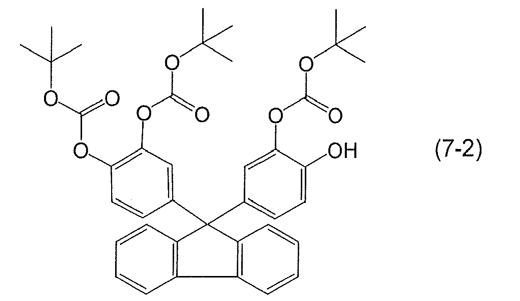

Into a solution prepared by mixing 1.91 g (5 mmol) of biscatecholfluorene (available form Osaka Gas Chemicals Co., Ltd.) with 5 ml of dimethylacetamide (DMAc), 3.27 g (15 mmol) of di-tert-butyl dicarbonate and 2.4 g of triethylamine were slowly added. The resultant mixture was stirred for 7 h at 60°C. The reaction liquid was separated and purified by a silica gel column chromatography (eluent: ethyl acetate/hexane = 1/4), and then dried under reduced pressure to obtain the titled compound. The structure of the compound was determined by elemental analysis and ¹H-NMR measurement. The results of the analysis are shown in Tables 1 and 2.

### EXAMPLE 13: Synthesis of Compound 7-3

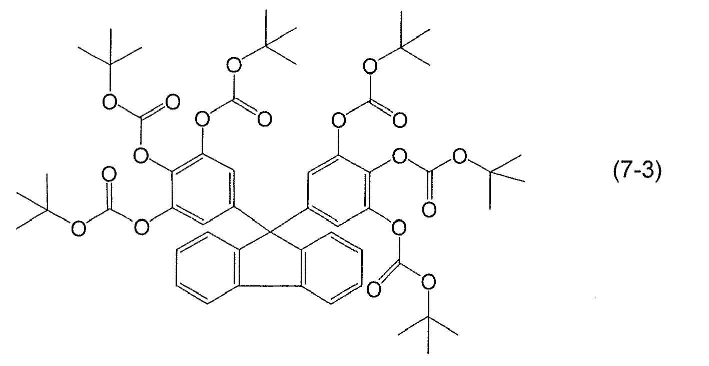

### (1) Synthesis of 9,9-bis(3,4,5-trihydroxyphenyl)fluorene

Into a solution prepared by heating a mixture of 50.4 g (0.4 mol) of pyrogallol (reagent available from Kanto Chemical Co., Inc.) and 18.0 g (0.1 mol) of 9-fluorenone (reagent available from Kanto Chemical Co., Inc.) to about 60°C, 0.1 ml of sulfuric acid, 0.8 ml of 3-mercaptopropionic acid and 10 ml of toluene were added. The reaction was allowed to proceed under stirring. After the conversion of 9-fluorenone reached 100%, 100 ml of toluene was added. The solid matters precipitated by cooling were collected by vacuum filtration, washed with a warm water of 60°C under stirring, and recrystallized, to obtain 4.30 g of the titled compound.

### (2) Synthesis of Compound 7-3

Into a solution prepared by mixing 0.103 g (0.25 mmol) of 9,9-bis(3,4,5-trihydroxyphenyl)fluorene synthesized above with 5 ml of anhydrous acetone and 1.2 mg of dimethylaminopyridine, 0.39 g (1.8 mmol) of di-tert-butyl dicarbonate was added dropwise over 30 min. The resultant mixture was stirred for 24 h at 40°C. The reaction liquid was poured into an excessive amount of water to precipitate solid matters. The obtained white powder was dried under reduced pressure, to obtain the titled compound. The structure of the compound was determined by elemental analysis and ¹H-NMR measurement. The results of the analysis are shown in Tables 1 and 2.

### EXAMPLE 14: Synthesis of Compound 7-4

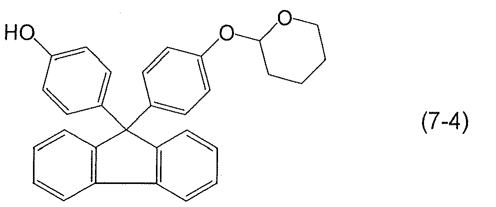

A solution prepared by mixing 1.75g (5 mmol) of bisphenolfluorene (available from Osaka Gas Chemicals Co., Ltd.) with 5 ml of anhydrous acetone, 0.073 g (0.29 mmol) of pyridinium p-toluenesulfonate and 0.50 g (6 mmol) of dihydropyran was stirred for 24 h at room temperature. The reaction liquid was purified by a silica gel column chromatography (eluent: ethyl acetate/hexane = 1/3) to obtain the titled compound. The structure of the compound was determined by elemental analysis and ¹H-NMR measurement. The results of the analysis are shown in Tables 1 and 2.

### EXAMPLE 15: Synthesis of Compound 7-5

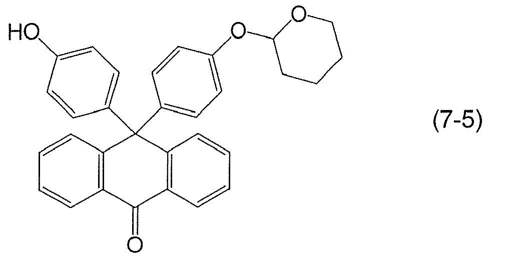

A solution prepared by mixing 1.89 g (5 mmol) of 10,10-bis(4-hydroxyphenyl)-9-anthrone (available from Honshu Chemical Industry Co., Ltd.) with 20 ml of anhydrous acetone, 0.073 g (0.29 mmol) of pyridinium p-toluenesulfonate and 0.50 g (6 mmol) of dihydropyran was stirred for 24 h at room temperature. The reaction liquid was purified by a silica gel column chromatography (eluent: ethyl acetate/hexane = 1/3) to obtain the titled compound. The structure of the compound was determined by elemental analysis and ¹H-NMR measurement. The results of the analysis are shown in Tables 1 and 2:

### EXAMPLE 16: Synthesis of Compound 8-1

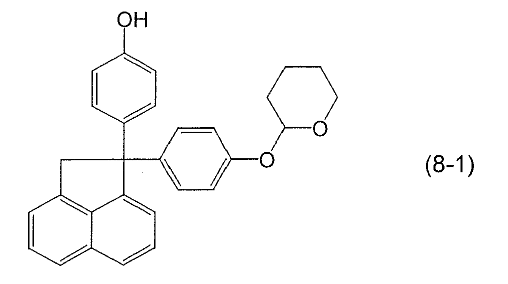

### (1) Synthesis of 1,1-bis(4-hydroxyphenyl)acenaphthene

Into a solution prepared by heating a mixture of 43.2 g (0.4 mol) phenol and 16.8 g (0.1 mol) of acenaphthenone to about 30°C, 0.1 ml of sulfuric acid, 0.8 ml of 3-mercaptopropionic acid and 10 ml of toluene were added. The reaction was allowed to proceed under stirring. After the conversion reached 100%, 100 ml of toluene was added. The solid matters precipitated by cooling were collected by vacuum filtration, washed with a warm water of 60°C under stirring, and recrystallized, to obtain the titled compound.

### (2) Synthesis of Compound 8-1

A solution prepared by mixing 1.69 g (5 mmol) of 1,1-bis(4-hydroxyphenyl)acenaphthene synthesized above with 10 ml of anhydrous acetone, 0.073 g (0.29 mmol) of pyridinium p-toluenesulfonate and 0.50 g (6 mmol) of dihydropyran was stirred for 24 h at room temperature. The reaction liquid was purified by a silica gel column chromatography (eluent: ethyl acetate/hexane = 1/3) to obtain the titled compound. The structure of the compound was determined by elemental analysis and ¹H-NMR measurement. The results of the analysis are shown in Tables 1 and 2.

### EXAMPLE 17: Synthesis of Compound 8-2

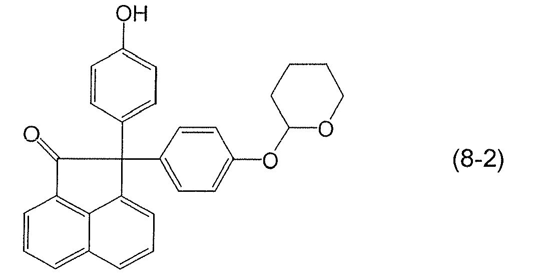

### (1) Synthesis of 1,1-bis(4-hydroxyphenyl)acenaphthene-2-one

In the same manner as in Example 16(1) except for using 16.2 g (0.1 mol) of acenaphthenequinone (available from Kanto Chemical Co., Inc.) in place of 16.8 g (0.1 mol) of acenaphthenone, 1,1-bis(4-hydroxyphenyl)acenaphthene-2-one was synthesized.

### (2) Synthesis of Compound 8-2

In the same manner as in Example 16(2) except for using 1.83 g (5 mmol) of 1,1-bis(4-hydroxyphenyl)acenaphthene-2-one in place of 1.69 g (5 mmol) of 1,1-bis(4-hydroxyphenyl)acenaphthene, the titled compound was synthesized. The structure of the compound was determined by elemental analysis and ¹H-NMR measurement. The results of the analysis are shown in Tables 1 and 2.

### EXAMPLE 18: Synthesis of Compound 8-3

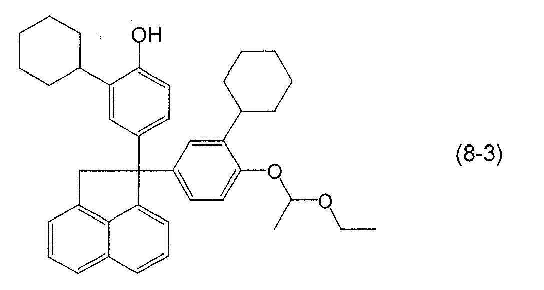

### (1) Synthesis of 1,1-bis(3-cyclohexyl-4-hydroxyphenyl)acenaphthene

In the same manner as in Example 16(1) except for using 53.0 g (0.3 mol) of 2-cyclohexylphenol in place of 43.2 g (0.4 mol) of phenol, 1,1-bis(3-cyclohexyl-4-hydroxyphenyl)acenaphthene was synthesized.

### (2) Synthesis of Compound 8-3

In the same manner as in Example 4 except for using 2.51 g (5 mmol) of 1,1-bis(3-cyclohexyl-4-hydroxyphenyl)acenaphthene in place of 1.84 g (5 mmol) of 1-(2-naphthyl)-1,1-bis(3-methyl-4-hydroxyphenyl)ethane, the titled compound was synthesized. The structure of the compound was determined by elemental analysis and ¹H-NMR measurement. The results of the analysis are shown in Tables 1 and 2.

### EXAMPLE 19: Synthesis of Compound 8-4

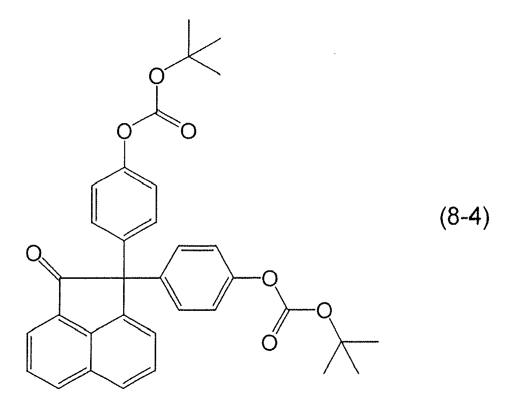

Into a solution prepared by mixing 1.76 g (5 mmol) of 1,1-bis(4-hydroxyphenyl)acenaphthene-2-one synthesized in Example 17(1) with 5 ml of anhydrous acetone and 1.2 mg of dimethylaminopyridine, 2.64 g (12 mmol) of di-tert-butyl dicarbonate was added dropwise over 10 min. The resultant mixture was stirred for 24 h at 40°C. The reaction liquid was poured into an excessive amount of water to precipitate solid matters. The obtained white powder was washed with distilled water three times, filtered under suction, and dried under reduced pressure, to obtain the titled compound. The structure of the compound was determined by elemental analysis and ¹H-NMR measurement. The results of the analysis are shown in Tables 1 and 2.

### EXAMPLE 20: Synthesis of Compound 8-5

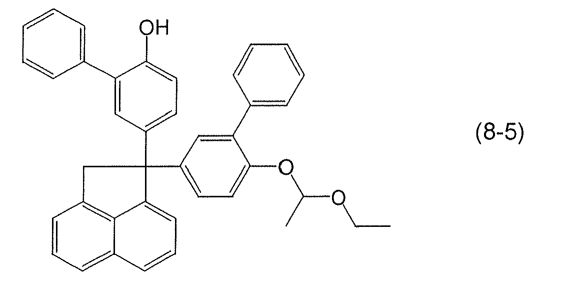

In the same manner as in Example 18 except for using 51.0 g (0.3 mol) of 2-phenylphenol in place of 53.0 g (0.3 mol) of 2-cyclohexylphenol, the titled compound was synthesized. The structure of the compound was determined by elemental analysis and ¹H-NMR measurement. The results of the analysis are shown in Tables 1 and 2.

### EXAMPLE 21: Synthesis of Compound 9-1

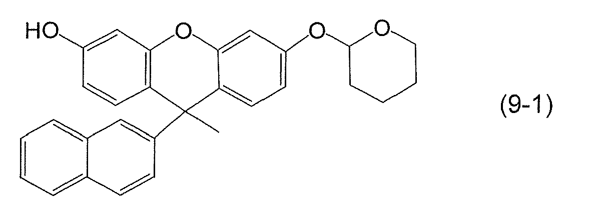

In the same manner as in Example 6 except for using 44.0 g (0.4 mol) of resorcinol in place of 43.2 g (0.4 mol) of o-cresol, the titled compound was synthesized. The structure of the compound was determined by elemental analysis and ¹H-NMR measurement. The results of the analysis are shown in Tables 1 and 2.

### EXAMPLE 22: Synthesis of Compound 10-1

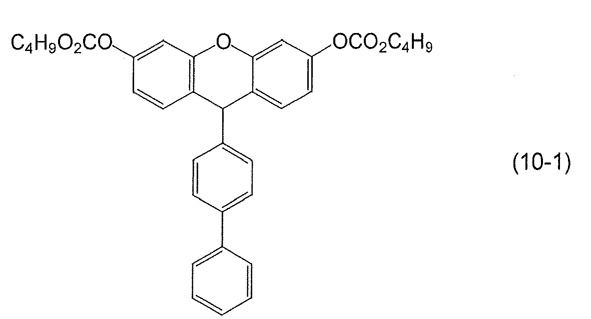

### (1) Synthesis of 2,8-dihydroxy-5-(4-biphenyl)xanthene

In the same manner as in Example 10(1) except for using 44.0 g (0.4 mol) of resorcinol in place of 37.4 g (0.4 mmol) of phenol, 2,8-dihydroxy-5-(4-biphenyl)xanthene was synthesized. The structure of the compound was determined by elemental analysis and ¹H-NMR measurement. The results of the analysis are shown in Tables 1 and 2.

### (2) Synthesis of Compound 10-1

In the same manner as in Example 19 except for using 1.83 g (5 mmol) of 2,8-dihydroxy-5-(4-biphenyl)xanthene in place of 1.76 g (5 mmol) of 1,1-bis(4-hydroxyphenyl)acenaphthene-2-one, the titled compound was synthesized. The structure of the compound was determined by elemental analysis and ¹H-NMR measurement. The results of the analysis are shown in Tables 1 and 2.

### EXAMPLE 23: Synthesis of Compound 10-2

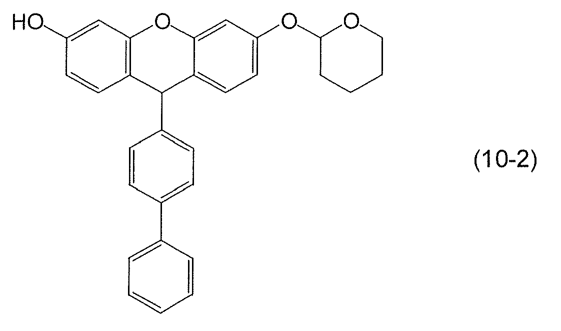

In the same manner as in Example 10 except for using 44.0 g (0.4 mol) of resorcinol in place of 37.4 g (0.4 mmol) of phenol, the titled compound was synthesized. The structure of the compound was determined by elemental analysis and ¹H-NMR measurement. The results of the analysis are shown in Tables 1 and 2.

### EXAMPLE 24: Synthesis of Compound 11-1

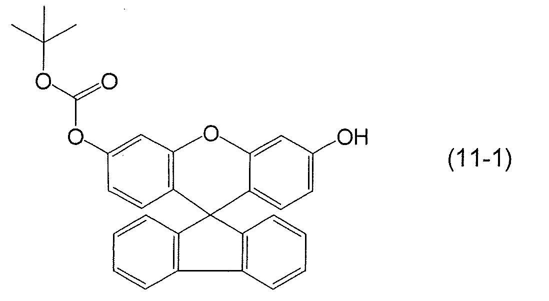

In the same manner as in Example 1 except for using 44.0 g (0.4 mol) of resorcinol in place of 43.2 g (0.4 mol) of o-cresol and using 18.0 g (0.1 mol) of 9-fluorene in place of 17.1 g (0.1 mol) of β-acetonaphthone, the titled compound was synthesized. The structure of the compound was determined by elemental analysis and ¹H-NMR measurement. The results of the analysis are shown in Tables 1 and 2.

### EXAMPLE 25: Synthesis of Compound 11-2

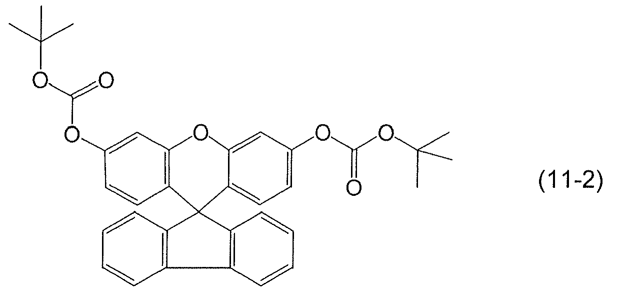

In the same manner as in Example 22 except for using 18.0 g (0.1 mol) of 9-fluorenone (reagent available from Kanto Chemical Co., Inc.) in place of 18.2 g (0.1 mol) of biphenylaldehyde, the titled compound was synthesized. The structure of the compound was determined by elemental analysis and ¹H-NMR measurement. The results of the analysis are shown in Tables 1 and 2.

### EXAMPLE 26: Synthesis of Compound 11-3

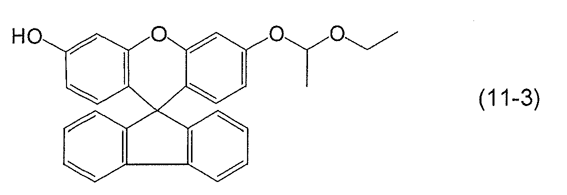

In the same manner as in Example 4 except for using 1.82 g (5 mmol) of 2,8-dihydroxy-5-(9,9-fluorenyl)xanthene synthesized in Example 24 in place of 1.84 g (5 mmol) of 1-(2-naphthyl)-1,1-bis(3-methyl-4-hydroxyphenyl)ethane, the titled compound was synthesized. The structure of the compound was determined by elemental analysis and ¹H-NMR measurement. The results of the analysis are shown in Tables 1 and 2.

### EXAMPLE 27: Synthesis of Compound 12-1

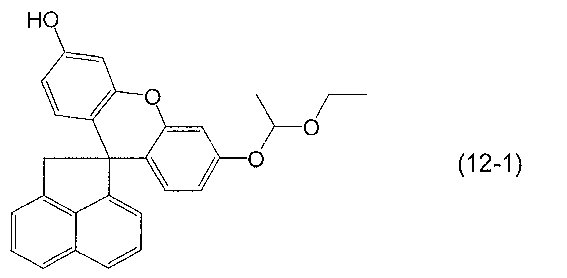

In the same manner as in Example 26 except for using 16.8 g (0.1 mol) of acenaphthenone in place of 18.0 g (0.1 mol) of 9-fluorenone, the titled compound was synthesized. The structure of the compound was determined by elemental analysis and ¹H-NMR measurement. The results of the analysis are shown in Tables 1 and 2.

### EXAMPLE 28: Synthesis of Mixture 13-1

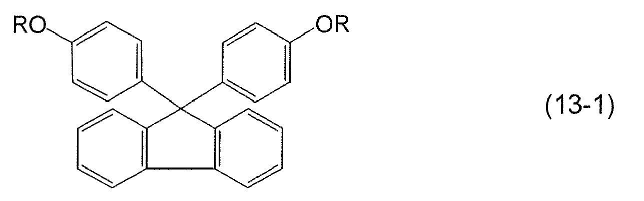

R = H or t-butoxycarbonyl group
Introduction of t-butoxycarbonyl group = 55%

Into a solution prepared by mixing 1.75 g (5 mmol) of bisphenolfluorene with 5 ml of anhydrous acetone and 1.2 mg of dimethylaminopyridine, 1.20 g (5.5 mmol) of di-tert-butyl dicarbonate was added dropwise over 10 min. The resultant mixture was stirred for 24 h at 40°C. The reaction liquid was purified by a silica gel column chromatography (eluent: ethyl acetate/hexane = 1/3) to obtain the titled mixture. The introduction of the acid-dissociating functional group into phenolic hydroxyl group was 55% when determined by ¹H-NMR. The results of the analysis are shown in Tables 1 and 2.

### EXAMPLE 29: Synthesis of Mixture 13-2

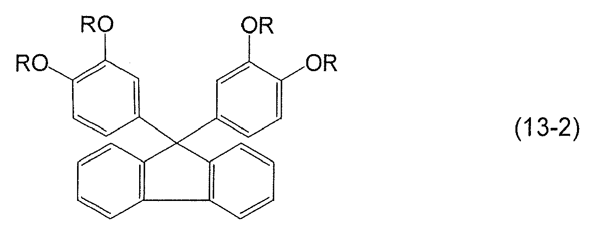

R = H or t-butoxycarbonyl group
Introduction of t-butoxycarbonyl group = 75%

Into a solution prepared by mixing 1.91 g (5 mmol) of biscatecholfluorene with 5 ml of anhydrous acetone and 1.2 mg of dimethylaminopyridine, 3.27 g (15 mmol) of di-tert-butyl dicarbonate was added dropwise over 10 min. The resultant mixture was stirred for 24 h at 40°C. The reaction liquid was purified by a silica gel column chromatography (eluent: ethyl acetate/hexane = 1/3) to obtain the titled mixture. The introduction of the acid-dissociating functional group into phenolic hydroxyl group was 75% when determined by ¹H-NMR. The results of the analysis are shown in Tables 1 and 2.

### EXAMPLE 30: Synthesis of Mixture 13-3

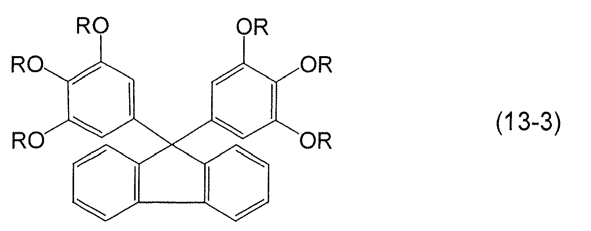

R = H or t-butoxycarbonyl group
Introduction of t-butoxycarbonyl group = 80%

Into a solution prepared by mixing 0.103 g (0.25 mmol) of 9,9-bis(3,4,5-trihydroxyphenyl)fluorene synthesized in Example 13(1) with 5 ml of anhydrous acetone and 1.2 mg of dimethylaminopyridine, 0.26 g (1.2 mmol) of di-tert-butyl dicarbonate was added dropwise over 30 min. The resultant mixture was stirred for 24 h at 40°C. The reaction liquid was poured into an excessive amount of water to precipitate solid matters. The obtained white powder was dried under reduced pressure to obtain the titled mixture. The introduction of the acid-dissociating functional group into phenolic hydroxyl group was 80% when determined by elemental analysis and ¹H-NMR. The results of the analysis are shown in Tables 1 and 2.

### EXAMPLE 31: Synthesis of Mixture 13-4

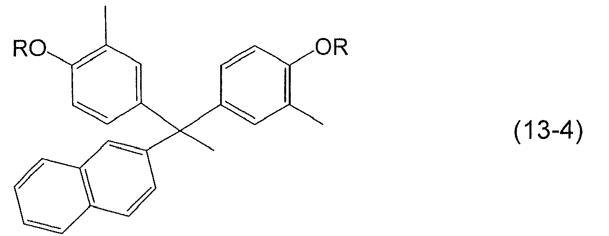

R = H or t-butoxycarbonyl group
Introduction of t-butoxycarbonyl group = 95%

Into a solution prepared by mixing 1.84 g (5 mmol) of 1-(2-naphthyl)-1,1-bis(3-methyl-4-hydroxyphenyl)ethane with 5 ml of anhydrous acetone and 1.2 mg of dimethylaminopyridine, 1.20 g (9.5 mmol) of di-tert-butyl dicarbonate was added dropwise over 10 min. The resultant mixture was stirred for 24 h at 40°C. The reaction liquid was purified by a silica gel column chromatography (eluent: ethyl acetate/hexane = 1/3) to obtain the titled mixture. The introduction of the acid-dissociating functional group into phenolic hydroxyl group was 95% when determined by ¹H-NMR. The results of the analysis are shown in Tables 1 and 2.

### EXAMPLE 32: Synthesis of Mixture 13-5

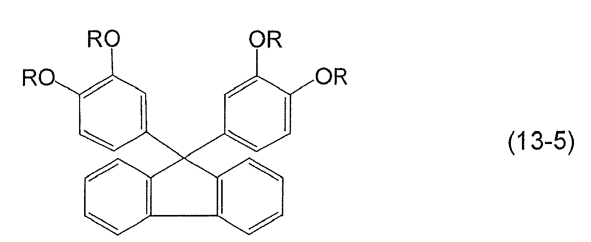

R = H or t-butoxycarbonyl group
Introduction of t-butoxycarbonyl group = 95%

Into a solution prepared by mixing 1.91 g (5 mmol) of biscatecholfluorene with 5 ml of anhydrous acetone and 1.2 mg of dimethylaminopyridine, 3.27 g (19 mmol) of di-tert-butyl dicarbonate was added dropwise over 10 min. The resultant mixture was stirred for 24 h at 40°C. The reaction liquid was purified by a silica gel column chromatography (eluent: ethyl acetate/hexane = 1/3) to obtain the titled mixture. The introduction of the acid-dissociating functional group into phenolic hydroxyl group was 95% when determined by ¹H-NMR. The results of the analysis are shown in Tables 1 and 2.

### COMPARATIVE EXAMPLE 1: Synthesis of Compound 14-1

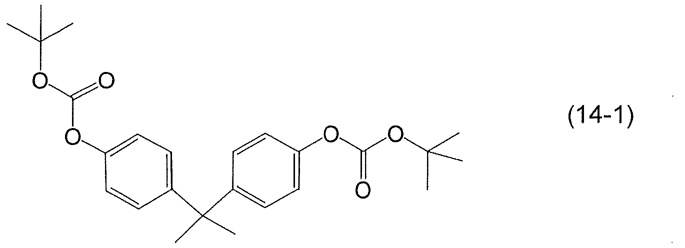

Into a solution prepared by mixing 1.14 g (5 mmol) of bisphenol A (reagent available from Kanto Chemical Co., Inc.) with 5 ml of anhydrous acetone and 1.2 mg of dimethylaminopyridine, 2.62 g (12 mmol) of di-tert-butyl dicarbonate was added dropwise over 10 min. The resultant mixture was stirred for 24 h at 40°C. The reaction liquid was poured into an excessive amount of water to precipitate solid matters. The obtained white powder was washed with distilled water three times, filtered under suction, and dried under reduced pressure, to obtain the titled compound. The structure of the compound was determined by elemental analysis and ¹H-NMR measurement. The results of the analysis are shown in Tables 1 and 2.

### COMPARATIVE EXAMPLE 2: Synthesis of Compound 14-2

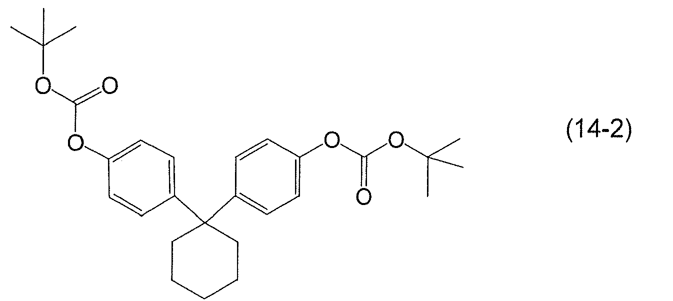

In the same manner as in Comparative Example 1 except for using 1.34 g (5 mmol) of bisphenol Z (reagent available from Kanto Chemical Co., Inc.) in place of 1.14 g (5 mmol) of bisphenol A, the titled compound was synthesized. The structure of the compound was determined by elemental analysis and ¹H-NMR measurement. The results of the analysis are shown in Tables 1 and 2.

### COMPARATIVE EXAMPLE 3: Synthesis of Compound 14-3

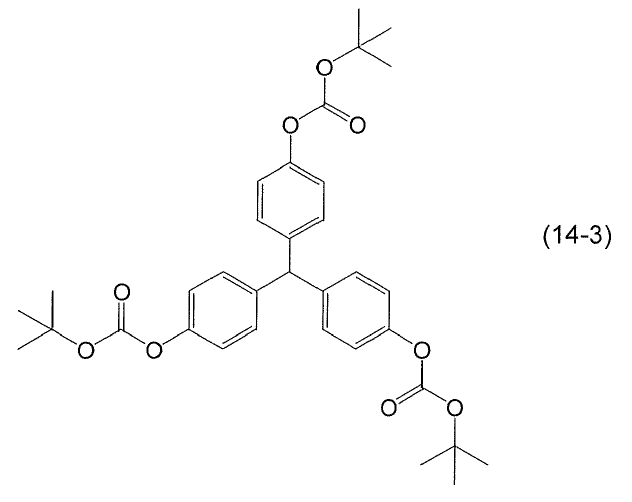

In the same manner as in Comparative Example 1 except for using 1.46 g (5 mmol) of tris(4-hydroxyphenyl)methane (available from Honshu Chemical Industry Co., Ltd.) in place of 1.14 g (5 mmol) of bisphenol A and changing the amount of di-tert-butyl dicarbonate from 2.62 g (12 mmol) to 3.93 g (16 mmol), the titled compound was synthesized. The structure of the compound was determined by elemental analysis and ¹H-NMR measurement. The results of the analysis are shown in Tables 1 and 2.

### COMPARATIVE EXAMPLE 4: Synthesis of Compound 14-4

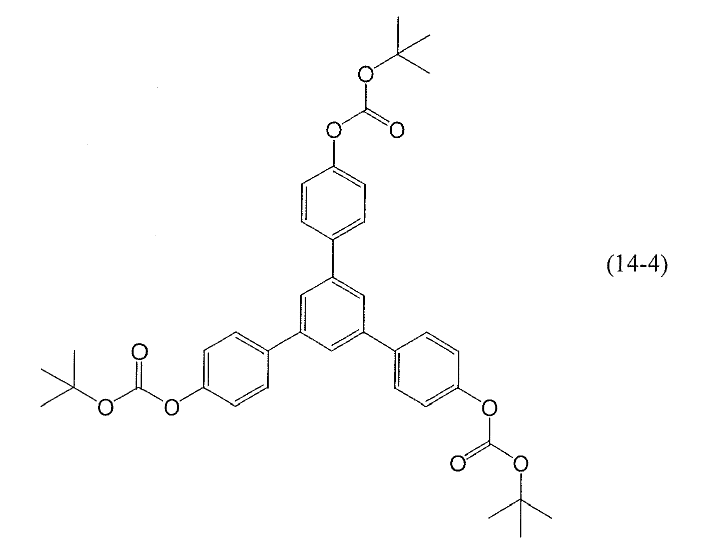

In the same manner as in Comparative Example 1 except for using 1.77 g (5 mmol) of tris(4-hydroxyphenyl)benzene (reagent available from Aldrich Chemical Co., Inc.) in place of 1.14 g (5 mmol) of bisphenol A and changing the amount of di-tert-butyl dicarbonate from 2.62 g (12 mmol) to 3.93 g (16 mmol), the titled compound was synthesized. The structure of the compound was determined by elemental analysis and ¹H-NMR measurement. The results of the analysis are shown in Tables 1 and 2.

### COMPARATIVE EXAMPLE 5

In the same manner as in Comparative Example 1 except for using 0.74 g (5 mmol) of polyhydroxystyrene (PHS-1) having a weight average molecular weight of 8000 (available from Aldrich Chemical Co., Inc.) in place of 1.14 g (5 mmol) of bisphenol A and changing the amount of di-tert-butyl dicarbonate from 2.62 g (12 mmol) to 0.37 g (1.5 mmol), a mixture (PHS-2) was synthesized. The introduction of the acid-dissociating functional group into phenolic hydroxyl group was 30% when determined by ¹H-NMR.

**Table 1**

| Compound No. | | Formula | Molecular weight | Calculated | Found |
|---|---|---|---|---|---|
| Examples | | | | | |
| 1 | 5-1 | C₃₆H₄₀O₆ | 568 | C:76.03, H:7.09, O:16.88 | C:76.14, H:7.00 |
| 2 | 5-2 | C₃₁H₃₂O₄ | 468 | C:79.46, H:6.88, O:13.66 | C:79.50, H:6.83 |
| 3 | 5-3 | C₄₄H₅₂O₁₂ | 772 | C:68.38, H:6.78, O:24.84 | C:68.51, H:6.71 |
| 4 | 5-4 | C₃₀H₃₂O₃ | 440 | C:81.78, H:7.32, O:10.89 | C:81.80, H:7.35 |
| 5 | 5-5 | C₃₄H₃₈O₃ | 494 | C:82.55, H:7.74, O:9.70 | C:82.70, H:7.60 |
| 6 | 5-6 | C₃₁H₃₂O₃ | 452 | C:82.27, H:7.13, O:10.61 | C:82.38, H:7.02 |
| 7 | 5-7 | C₄₀H₄₈O₃ | 576 | C:83.29, H:8.39, O:8.32 | C:83.41, H:8.30 |
| 8 | 5-8 | C₂₉H₂₈O₄ | 440 | C:79.07, H:6.41, O:14.53 | C:79.16, H:6.31 |
| 9 | 5-9 | C₂₈H₂₆O₄ | 426 | C:78.83, H:6.14, O:15.01 | C:78.95, H:6.04 |
| 10 | 6-1 | C₃₀H₂₈O₃ | 436 | C:82.54, H:6.46, O:11.00 | C:82.50, H:6.53 |
| 11 | 7-1 | C₄₅H₅₀O₁₂ | 782 | C:69.04, H:6.44, O:24.54 | C:69.13, H:6.38 |
| 12 | 7-2 | C₄₀H₄₂O₁₀ | 682 | C:70.37, H:6.20, O:23.43 | C:70.45, H:6.04 |
| 13 | 7-3 | C₅₅H₆₆O₁₈ | 1014 | C:65.08, H:6.55, O:28.37 | C:65.18, H:6.65 |
| 14 | 7-4 | C₃₀H₂₆O₃ | 434 | C:82.92, H:6.03, O:11.05 | C:83.01, H:6.00 |
| 15 | 7-5 | C₃₁H₂₆O₄ | 462 | C:80.50, H:5.67, O:13.84 | C:80.40, H:5.77 |
| 16 | 8-1 | C₂₉H₂₆O₃ | 422 | C:82.44, H:6.20, O:11.36 | C:82.42, H:6.22 |
| 17 | 8-2 | C₂₉H₂₄O₄ | 436 | C:79.80, H:5.54, O:1.4.66 | C:79.91, H:5.50 |
| 18 | 8-3 | C₄₀H₄₆O₃ | 574 | C:83.58, H:8.07, O:8.35 | C:83.40, H:8.27 |
| 19 | 8-4 | C₃₄H₃₂O₇ | 552 | C:73.90, H:5.84, O:20.27 | C:73.81, H:5.80 |
| 20 | 8-5 | C₄₀H₃₄O₃ | 528 | C:83.38, H:6.09, 0:8.53 | C:83.42, H:6.02 |
| 21 | 9-1 | C₂₉H₂₆O₄ | 438 | C:79.43, H:5.98, O:14.59 | C:79.53, H:5.91 |
| 22 | 10-1 | C₃₅H₃₄O₇ | 566 | C:74.19, H:6.05, O:19.76 | C:74.11, H: 6.15 |
| 23 | 10-2 | C₃₀H₂₆O₄ | 450 | C:79.98, H:5.82, 0:14.21 | C:80.05, H:5.72 |
| 24 | 11-1 | C₃₀H₂₄O₅ | 464 | C:77.57, H:5.21, O:17.22 | C:77.64, H:5.16 |
| 25 | 11-2 | C₃₅H₃₂O₇ | 564 | C:74.45, H:5.71, O:19.84 | C:74.55, H:5.61 |
| 26 | 11-3 | C₂₉H₂₄O₄ | 436 | C:79.80, H:5.54, O:14.66 | C:79.63, H:5.67 |
| 27 | 12-1 | C₂₈H₂₄O₄ | 424 | C:79.22, H:5.70, O:15.08 | C:79.16, H:5.80 |
| 28 | 13-1 | - | - | - | - |
| 29 | 13-2 | - | - | - | - |
| 30 | 13-3 | - | - | - | - |
| 31 | 13-4 | - | - | - | - |
| 32 | 13-5 | - | - | - | - |

| Comparative Examples | | | | | |
|---|---|---|---|---|---|
| 1 | 14-1 | C₂₅H₃₂O₆ | 428 | C:70.07, H:7.53, O:22.40 | C:70.0, H:7.61 |
| 2 | 14-2 | C₂₈H₃₆O₆ | 469 | C:71.77, H:7.74, O:20.49 | C:71.73, H:7.62 |
| 3 | 14-3 | C₃₄H₄₀O₉ | 592 | C:68.97, H:6.80, O:24.3 | C:69.2, H:6.71 |
| 4 | 14-4 | C₃₉H₄₂O₉ | 654 | C:71.54, H:6.47, 0:21.99 | C:71.73, H:6.25 |

**Table 2**

| Compound No. | | ¹H-NMR δ (ppm) |
|---|---|---|
| Examples | | |
| 1 | 5-1 | 7.6-6.8 (13H, m), 2.1 (9H, d), 1.4 (18H, s) |
| 2 | 5-2 | 7.8-6.6 (13H, m), 4.7 (1H, br), 2.2 (9H, t), 1.5 (9H, s) |
| 3 | 5-3 | 7.8-7.3 (13H, m), 2.3 (3H, s), 1.5 (36H) |
| 4 | 5-4 | 7.8-6.6 (13H, m), 5.4 (1H, m), 4.7 (1H, br), 3.7-3.4 (2H, m), 2.2 (9H, t), 1.4 (3H, s), 1.1 (3H, t) |
| 5 | 5-5 | 7.8-6.6 (13H, m), 5.5 (1H, s), 3.5 (1H, br), 2.3-1.5 (22H, m) |
| 6 | 5-6 | 7.8-6.6 (13H, m), 5.5 (1H, s), 4.7 (1H, br), 3.7-3.5 (2H, m), 2.2 (9H, t), 2.1-1.8 (6H, m) |
| 7 | 5-7 | 7.8-6.5 (13H, m), 5.2 (1H, s), 4.9 (1H, br), 3.5 (2H, q), 2.7 (2H, m), 2.3 (3H, s), 1.7-1.2 (26H, m) |
| 8 | 5-8 | 7.8-6.6 (15H, m), 4.7 (1H, br), 2.2 (3H, s), 1.5 (9H, s) |
| 9 | 5-9 | 7.9-6.5 (15H, m), 4.9 (1H, br), 1.5 (9H, s) |
| 10 | 6-1 | 7.6-7.0 (17H, m), 5.6 (1H, s), 5.5 (1H, s), 5.4 (1H, s), 3.8-3.6 (2H, m), 2.1-1.8 (6H, m) |
| 11 | 7-1 | 7.9-7.1 (14H, m), 2.1 (3H, s), 1.5 (36H, s) |
| 12 | 7-2 | 7.9-7.1 (14H, m), 5.5 (1H, br), 2.1 (3H, s), 1.5 (27H, s) |
| 13 | 7-3 | 7.7-7.3 (8H, s), 6.9 (4H, s), 1.5 (54H, s) |
| 14 | 7-4 | 8.1-7.0 (16H, m), 5.5 (1H, s), 5.4 (1H, br), 3.8-3.6 (2H, m), 2.1-1.8 (6H, m) |
| 15 | 7-5 | 8.1-6.6 (16H, m), 5.5 (1H, s), 5.4 (1H, br), 3.8-3.6 (2H, m), 2.1-1.8 (6H, m) |
| 16 | 8-1 | 7.7-6.8 (14H, m), 5.5 (1H, s), 5.4 (1H.s), 4,1-3.9 (2H, q), 3.8-3.6 (2H, m), 2.1-1.8 (6H, m) |
| 17 | 8-2 | 8.2-6.9 (14H, m), 5.5 (1H.s), 5.4 (1H, br), 3.8-3.6 (2H, m), 2.1-1.8 (6H, m) |
| 18 | 8-3 | 7.7-6.5 (12H, m), 5.2 (1H, s), 4.9 (1H, br), 4.1-3.8 (2H, q), 3.5 (2H, q), 2.8-2.5 (2H, m), 1.7-1.2 (16H, m) |
| 19 | 8-4 | 8.2-6.9 (14H, m), 1.4 (18H, s) |
| 20 | 8-5 | 7.7-6.9 (22H, m), 5.5 (1H, q), 5.3 (1H, br), 4.1-3.8 (2H, q), 3.5 (2H, q), 1.6 (3H, d), 1.2 (3H, t) |
| 21 | 9-1 | 7.9-7.2 (7H, m), 6.8-6.6 (4H, q), 6.4 (2H, d), 5.4 (1H, s), 3.7 (2H, t), 2.4 (3H, s), 2.1-1.8 (6H, m) |
| 22 | 10-1 | 7.7-7.0 (13H, m), 6.8 (2H, s), 5.4 (1H, s), 1.5 (18H, s) |
| 23 | 10-2 | 7.7-7.2 (9H, m), 6.9--6.4 (6H, m), 6.2 (1H, s), 5.5 (1H, s), 5.4 (1H, s), 3.9-3.7 (2H, t), 2.1-1.8 (6H, m) |
| 24 | 11-1 | 8.2-6.5 (14H, m), 6.2 (1H, br), 1.5 (9H, s) |
| 25 | 11-2 | 8.2-7.0 (14H, m), 1.5 (18H, s), 1.2 (3H, t) |
| 26 | 11-3 | 8.2-6.5 (14H, m), 6.2 (1H, br), 3.5 (2H, q), 1.6 (3H, d), |
| 27 | 12-1 | 7.7-6.4 (12H, m), 6.2 (1H, br), 5.3 (1H, s), 3.8-3.6 (2H, q), 3.5-3.4 (2H, q), 1.6 (3H, d), 1.2 (3H, t) |
| 28 | 13-1 | 8.0-7.0 (17H, m), 1.5 (10H, s) |
| 29 | 13-2 | 7.9-7.1 (14H, m), 5.5 (1H, br), 2.1 (3H, s), 1.5 (27H, s) |
| 30 | 13-3 | 7.7-7.3 (8H, s), 6.9 (4H, s), 5.5 (1H, br), 1.5 (43H, s) |
| 31 | 13-4 | 7.6-6.8 (13H, m), 2.1 (9H, d), 1.4 (17H, s) |
| 32 | 13-5 | 7.9-7.1 (14H, m), 5.5 (1H, br), 2.1 (3H, s), 1.5 (34H, s) |

| Comparative Examples | | |
|---|---|---|
| 1 | 14-1 | 7.3 (2H, d), 7.1 (2H, d), 2.2 (4H, s), 1.5 (18H, s) |
| 2 | 14-2 | 7.3 (2H, d), 7.1 (2H, d), 2.2-1.5 (28H, m) |
| 3 | 14-3 | 7.3 (12H, s), 1.5 (27H, s) |
| 4 | 14-4 | 7.7 (9H, m), 7.3 (6H, d), 1.5 (27H, m) |

### EXAMPLES 33-64 and COMPARATIVE EXAMPLES 5-9: Properties of Compounds and Radiation Sensitive Compositions

The compounds and compositions obtained in Examples 1-32 and Comparative Examples 1-4 were tested for their properties. The results are shown in Tables 3 and 4.

### EXAMPLE 65

A mixture consisting of 50 mol % of Compound 5-6 and 50 mol % of its intermediate product, 1-(2-naphthyl)-1,1-bis(3-methyl-4-hydroxyphenyl)ethane (Compound 14-5), was tested for its properties. The number of the substituent OR¹ was 25% of the total number of the substituents OR¹ and OH. The results are shown in Tables 3 and 4.

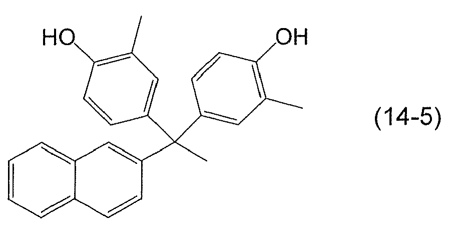

### EXAMPLE 66

A mixture consisting of 40 mol % of Compound 5-7 and 60 mol % of its intermediate product, 1-(2-naphthyl)-1,1-bis(3-cyclohexyl-4-hydroxyphenyl)ethane (Compound 14-6), was tested for its properties. The number of the substituent OR¹ was 20% of the total number of the substituents OR¹ and OH. The results are shown in Tables 3 and 4.

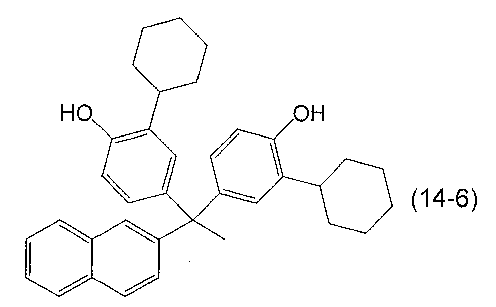

### COMPARATIVE EXAMPLE 9

Compound 14-5 was tested for its properties. The results are shown in Tables 3 and 4.

**Table 3**

| Compound No. | | (1) Solubility in safety solvent | (2) Film-forming properties | (3) Inhibition of dissolution in developing solution |
|---|---|---|---|---|
| Examples | | | | |
| 33 | 5-1 | A | A | A |
| 34 | 5-2 | A | A | A |
| 35 | 5-3 | A | A | A |
| 36 | 5-4 | A | A | A |
| 37 | 5-5 | A | A | A |
| 38 | 5-6 | A | A | A |
| 39 | 5-7 | A | A | A |
| 40 | 5-8 | A | A | A |
| 41 | 5-9 | A | A | A |
| 42 | 6-1 | A | A | A |
| 43 | 7-1 | B | B | A |
| 44 | 7-2 | A | A | A |
| 45 | 7-3 | A | A | A |
| 46 | 7-4 | A | A | A |
| 47 | 7-5 | A | A | A |
| 48 | 8-1 | A | A | A |
| 49 | 8-2 | A | A | A |
| 50 | 8-3 | A | A | A |
| 51 | 8-4 | A | A | A |
| 52 | 8-5 | A | A | A |
| 53 | 9-1 | A | A | A |
| 54 | 10-1 | A | A | A |
| 55 | 10-2 | A | A | A |
| 56 | 11-1 | A | A | A |
| 57 | 11-2 | B | B | A |
| 58 | 11-3 | A | A | A |
| 59 | 12-1 | A | A | A |
| 60 | 13-1 | A | A | A |
| 61 | 13-2 | A | A | A |
| 62 | 13-3 | A | A | A |
| 63 | 13-4 | A | A | A |
| 64 | 13-5 | B | B | A |
| 65 | (5-6)/(14-5) = 50/50 | A | A | A |
| 66 | (5-7)/(14-6) = 40/60 | A | A | A |

| Comparative Examples | | | | |
|---|---|---|---|---|
| 5 | 14-1 | C | - | - |
| 6 | 14-2 | C | - | - |
| 7 | 14-3 | A | A | A |
| 8 | 14-4 | A | B | A |
| 9 | 14-5 | A | A | C |

**Table 4**

| Compound No. | | (4) Adhesion to Si substrate | (5) Alkali developability |
|---|---|---|---|
| Examples | | | |
| 33 | 5-1 | A | A |
| 34 | 5-2 | A | A |
| 35 | 5-3 | B | A |
| 36 | 5-4 | A | A |
| 37 | 5-5 | A | A |
| 38 | 5-6 | A | A |
| 39 | 5-7 | A | A |
| 40 | 5-8 | A | A |
| 41 | 5-9 | A | A |
| 42 | 6-1 | A | A |
| 43 | 7-1 | B | B |
| 44 | 7-2 | A | A |
| 45 | 7-3 | A | A |
| 46 | 7-4 | A | A |
| 47 | 7-5 | A | A |
| 48 | 8-1 | A | A |
| 49 | 8-2 | A | A |
| 50 | 8-3 | A | A |
| 51 | 8-4 | A | A |
| 52 | 8-5 | A | A |
| 53 | 9-1 | A | A |
| 54 | 10-1 | B | A |
| 55 | 10-2 | A | A |
| 56 | 11-1 | A | A |
| 57 | 11-2 | B | A |
| 58 | 11-3 | A | A |
| 59 | 12-1 | A | A |
| 60 | 13-1 | A | A |
| 61 | 13-2 | A | A |
| 62 | 13-3 | A | A |
| 63 | 13-4 | A | A |
| 64 | 13-5 | B | A |
| 65 | (5-6)/(14-5) = 50/50 | A | A |
| 66 | (5-7)/(14-6) = 40/60 | A | A |

| Comparative Examples | | | |
|---|---|---|---|
| 5 | 14-1 | - | A |
| 6 | 14-2 | - | A |
| 7 | 14-3 | C | A |
| 8 | 14-4 | C | A |
| 9 | 14-5 | - | A |

### EXAMPLES 67-88 and COMPARATIVE EXAMPLES 10-16: Evaluation of resist pattern and dry-etching resistance

Each of the compounds obtained in Examples 1-32 was formulated as shown in Table 5, and the resultant formulation was filtered through a 0.2 µm Teflon (registered trademark) filter to prepare a radiation sensitive composition. The resist pattern formed from the radiation sensitive composition was evaluated for its resolution and sensitivity. The results are shown in Table 6. In any of Examples 67-88, a good pattern with an extremely small edge roughness was obtained. Also, the amount of outgas during exposure was small.

Using each of the radiation sensitive compositions of Examples 72 and 86, a resist film with 100 nm thick was formed on a gallium-arsenic wafer. The resist film was dry-etched by a tetrafluoromethane etching gas for etching times of 30 s, 60 s, 90 s and 120 s using an RIE etching apparatus under etching conditions of 70 sccm, 50 W, and 20 Pa. The thicknesses for respective etching times were measured by a thickness measuring apparatus. The etching rate of each resist film was determined by the slope of an approximated line which fits the measured values. The etching rate was 8.8 nm/min in Example 72, and 20 nm/min in Example 86, showing high etching resistance.

**Table 5**

| Resist compound or composition (g) | | Resin (g) | Acid generator (g) | Acid-diffusion controller (g) | Solvent (g) |
|---|---|---|---|---|---|
| Examples | | | | | |
| 67 | 5-1 (0.5) | - | PAG-1 (0.05) | Q-1 (0.005) | S-1/S-2 (1.3/3.2) |
| 68 | 5-2 (0.5) | - | PAG-1 (0.05) | Q-1 (0.005) | S-1/S-2 (1.3/3.2) |
| 69 | 5-2 (0.5) | - | PAG-2 (0.05) | Q-2 (0.005) | S-1/S-2 (1.3/3.2) |
| 70 | 5-2 (0.5) | PHS-1 (0.1) | PAG-2 (0.05) | Q-2 (0.005) | S-1/S-2 (1.3/3.2) |
| 71 | 5-2 (0.5) | PHS-2 (0.1) | PAG-2 (0.05) | Q-2 (0.005) | S-1/S-2 (1.3/3.2) |
| 72 | 5-5 (0.5) | - | PAG-2 (0.05) | Q-2 (0.005) | S-1/S-2 (1.3/3.2) |
| 73 | 5-6 (0.5) | - | PAG-1 (0.05) | Q-1 (0.005) | S-1/S-2 (1.3/3.2) |
| 74 | 5-6 (0.5) | - | PAG-2 (0.05) | Q-2 (0.005) | S-1/S-2 (1.3/3.2) |
| 75 | 6-1 (0. 5) | - | PAG-1 (0.05) | Q-1 (0.005) | S-1/S-2 (1.3/3.2) |
| 76 | 7-2 (0.5) | - | PAG-1 (0.05) | Q-1 (0.005) | S-1/S-2 (1.3/3.2) |
| 77 | 7-2 (0.5) | - | PAG-1 (0.05) | Q-1 (0.005) | S-1/S-2 (1.3/3.2) |
| 78 | 7-3 (0.5) | - | PAG-1 (0.05) | Q-1 (0.005) | S-1/S-2 (1.3/3.2) |
| 79 | 7-3 (0.5) | PHS-1 (0.1) | PAG-1 (0.05) | Q-1 (0.005) | S-1/S-2 (1.3/3.2) |
| 80 | 8-2 (0.5) | - | PAG-1 (0.05) | Q-1 (0.005) | S-1/S-2 (1.3/3.2) |
| 81 | 8-3 (0.5) | - | PAG-1 (0.05) | Q-1 (0.005) | S-1/S-2 (1.3/3.2) |
| 82 | 9-1 (0.5) | - | PAG-1 (0.05) | Q-1 (0.005) | S-1/S-2 (1.3/3.2) |
| 83 | 10-2 (0.5) | - | PAG-1 (0.05) | Q-1 (0.005) | S-1/S-2 (1.3/3.2) |
| 84 | 11-3 (0.5) | - | PAG-1 (0.05) | Q-1 (0.005) | S-1/S-2 (1.3/3.2) |
| 85 | 13-2 (0.5) | - | PAG-1 (0.05) | Q-1 (0.005) | S-1/S-2 (1.3/3.2) |
| 86 | 13-2 (0.5) | - | PAG-1/PAG-2 (0.02/0.03) | Q-1 (0.0005) | S-1/S-2 (1.3/3.2) |
| 87 | (5-6)/(14-5) 50/50 (0.5) | - | PAG-1 (0.05) | Q-1 (0.005) | S-1/S-2 (1.3/3.2) |
| 88 | (5-7)/(14-6) 40/60 (0.5) | - | PAG-1 (0.05) | Q-1 (0.005) | S-1/S-2 (1.3/3.2) |

| Comparative Examples | | | | | |
|---|---|---|---|---|---|
| 10 | 14-3 (0.5) | - | PAG-1 (0.05) | Q-1 (0.005) | S-1/S-2 (1.3/3.2) |
| 11 | 14-3 (0.5) | - | PAG-2 (0.05) | Q-2 (0.005) | S-1/S-2 (1.3/3.2) |
| 12 | 5-1 (0.2) | PHS-1 (0.3) | PAG-2 (0.05) | Q-2 (0.005) | S-1/S-2 (1.3/3.2) |
| 13 | 5-2 (0.2) | PHS-1 (0.3) | PAG-2 (0.05) | Q-2 (0.005) | S-1/S-2 (1.3/3.2) |
| 14 | 5-1 (0.2) | PHS-2 (0.3) | PAG-2 (0.05) | Q-2 (0.005) | S-1/S-2 (1.3/3.2) |
| 15 | 7-3 (0.2) | PHS-1 (0.3) | PAG-2 (0.05) | Q-2 (0.005) | S-1/S-2 (1.3/3.2) |
| 16 | 7-3 (0.2) | PHS-2 (0.3) | PAG-2 (0.05) | Q-2 (0.005) | S-1/S-2 (1.3/3.2) |

| | | | | | |
|---|---|---|---|---|---|
| PAG-1: diphenyltolylsulfonium nanofluorobutanesulfonate | | | | | |
| PAG-2: triphenylsulfonium trifluoromethanesulfonate | | | | | |
| Q-1: trioctylamine | | | | | |
| Q-2: diazabicyclooctane | | | | | |
| S-1: PGMEA | | | | | |
| S-2: EL | | | | | |

**Table 6**

| | PEB (°C) | Sensitivity (µC/cm²) | Resolution (nm) |
|---|---|---|---|
| Examples | | | |
| 67 | 100 | 40 | 50 |
| 68 | 80 | 20 | 50 |
| 69 | 80 | 20 | 50 |
| 70 | 80 | 10 | 60 |
| 71. | 80 | 10 | 60 |
| 72 | 80 | 10 | 50 |
| 73 | 80 | 10 | 50 |
| 74 | 80 | 10 | 50 |
| 75 | 80 | 10 | 50 |
| 76 | 100 | 20 | 50 |
| 77 | 100 | 20 | 50 |
| 78 | 100 | 30 | 50 |
| 79 | 80 | 10 | 60 |
| 80 | 80 | 10 | 50 |
| 81 | 80 | 10 | 50 |
| 82 | 80 | 10 | 50 |
| 83 | 80 | 10 | 50 |
| 84 | 80 | 10 | 50 |
| 85 | 80 | 20 | 50 |
| 86 | 80 | 20 | 50 |
| 87 | 80 | 10 | 50 |
| 88 | 80 | 5 | 50 |

| Comparative Examples | | | |
|---|---|---|---|
| 10 | 80 | 60 | 100 |
| 11 | 80 | 60 | 100 |
| 12 | 80 | 20 | 80 |
| 13 | 80 | 20 | 80 |
| 14 | 80 | 20 | 80 |
| 15 | 80 | 20 | 80 |
| 16 | 80 | 20 | 80 |

### EXAMPLE 89: Dry Etching Resistance

Using each of the radiation sensitive compositions of Examples 72 and 86, a resist film with 100 nm thick was formed on a gallium-arsenic wafer. The resist film was dry-etched by a tetrafluoromethane etching gas for etching times of 30 s, 60 s, 90 s and 120 s using an RIE etching apparatus under etching conditions of 70 sccm, 50 W, and 20 Pa. The thicknesses for respective etching times were measured by a thickness measuring apparatus. The etching rate of each resist film was determined by the slope of an approximated line which fits the measured values. The etching rate was 8.8 nm/min in Example 72, and 20 nm/min in Example 86, showing high etching resistance.

### Industrial Applicability

The radiation sensitive compounds of the invention are used as a main ingredient of non-polymeric radiation sensitive compositions which are free from metal catalyst and excellent in sensitivity, resolution, heat resistance, etching resistance and solubility in solvent. The use of the radiation sensitive composition and the solubility improver in combination provides patterns with high resolution and high sensitivity, and therefore, highly integrated semiconductor devices can be produced in a high productivity.

## Claims

1. A radiation sensitive composition comprising from 1 to 80% by weight of a solid component and from 20 to 99% by weight of a solvent, the composition containing a compound B satisfying the following requirements a and b:
(a) having a structure formed by introducing an acid-dissociating functional group into at least one phenolic hydroxyl group of a polyphenol compound A that is produced by a condensation reaction of an aromatic ketone or an aromatic aldehyde each having from 5 to 45 carbon atoms with a compound having from 6 to 15 carbon atoms and from 1 to 3 phenolic hydroxyl groups, and
(b) having a molecular weight of from 300 to 3,000;
and a total content of the compound B and a solubility improver being from 50 to 99.999% by weight of a total weight of the solid component.

2. The radiation sensitive composition according to claim 1, wherein the compound B has a conjugated structure comprising at least two benzene rings and/or a nonbonding electron pair of hetero atom.

3. The radiation sensitive composition according to claim 2, wherein the conjugated structure is at least one structure selected from the group consisting of biphenyl structure, naphthalene structure, anthracene structure, phenanthrene structure, pyrene structure, fluorene structure, acenaphthene structure, 1-ketoacenaphthene structure, benzophenone structure, xanthene structure, and thioxanthene structure.

4. The radiation sensitive composition according to any one of claims 1 to 3, wherein the solid component contains at least one acid generator which generates acid upon irradiation of radiations such as extreme ultraviolet rays, electron beams and X-rays.

5. The radiation sensitive composition according to any one of claims 1 to 3, wherein the compound B is at least one compound selected from the group consisting of the compounds represented by the following formula 1: 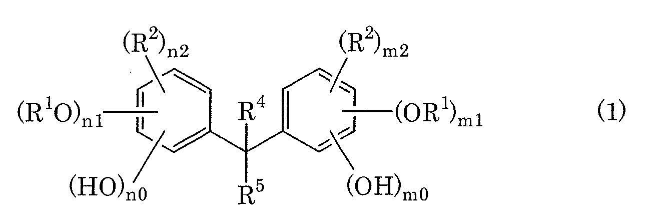 wherein each of R¹ groups is independently an acid-dissociating functional group selected from the group consisting of substituted methyl group, 1-substituted ethyl group, 1-substituted-n-propyl group, 1-branched alkyl group, silyl group, acyl group, 1-substituted alkoxymethyl group, cyclic ether group, and alkoxycarbonyl group;
each of R² group is independently a group selected from the group consisting of halogen atom, alkyl group, cycloalkyl group, aryl group, aralkyl group, alkoxy group, aryloxy group, alkyenyl group, acyl group, alkoxycarbonyl group, alkyloyloxy group, aryloyloxy group, cyano group, and nitro group;
R⁴ is hydrogen atom, C₁₋₆ alkyl group or aryl group, R⁵ is a monovalent Cio-is group having a biphenyl structure or a naphthalene structure, or -CR⁴R⁵⁻may be a bivalent C₁₀₋₁₈ group having a fluorene structure, a acenaphthene structure, 1-ketoacenaphthene structure or a benzophenone structure when R⁴ and R⁵ are bonded to each other;
m0 and n0 are each an integer of from 0 to 3, m1 and n1 are each an integer of from 0 to 3, and m2 and n2 are each an integer of from 0 to 4, each satisfying the formulae: 1 ≤ m0 + ml + m2 ≤ 5, 1 ≤ n0 + n1 + n2 ≤ 5, 1 ≤ ml + n1 ≤ 6, 1 ≤ m0 + m1 ≤ 3, and 1 ≤ n0 + n1 ≤ 3; and
two carbon atoms of two benzene rings each being at ortho-position with respect to -CR⁴R⁵ may be bonded to each other via oxygen atom or sulfur atom to form a xanthene structure or a thioxanthene structure represented by the following formula 2: 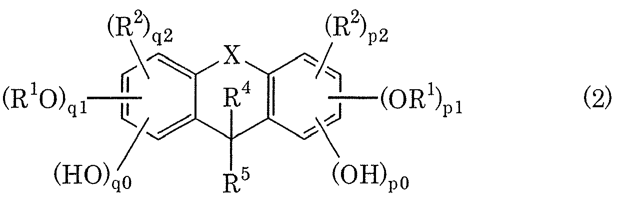 wherein R¹, R², R⁴ and R⁵ are the same as defined above; p0 and q0 are each an integer of from 0 to 2, p1 and q1 are each an integer of from 0 to 2, p2 and q2 are each an integer of from 0 to 3, each satisfying 1 ≤ p0 + p1+p2 ≤ 4, 1 ≤ q0 + q1 + q2 ≤ 4, 1 ≤ p1 + q1 ≤ 4, 1 ≤ p0 + p1 ≤ 2, and 1 ≤ q0 + q1 ≤ 2; and X is oxygen atom or sulfur atom.

6. The radiation sensitive composition according to claim 5, wherein R⁵ is represented by the following formula: 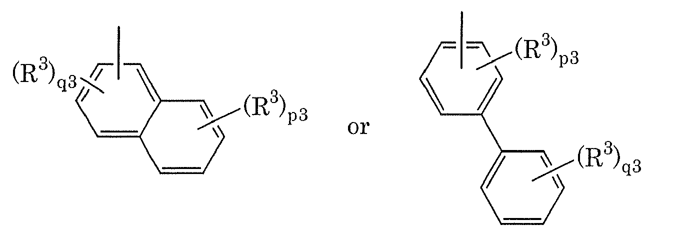 wherein R³ is a hydrogen atom or a C₁₋₆ alkyl group; p3 is an integer of from 0 to 4; q3 is an integer of from 0 to 3; and p3 and q3 satisfies 0 ≤ p3 + q3≤ 7.

7. The radiation sensitive composition according to claim 5, wherein R⁴ and R⁵ of -CR⁴R⁵- are bonded to each other such that -CR⁴R⁵- forms a bivalent group represented by the following formula: 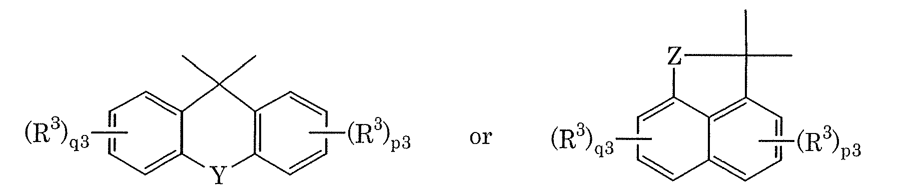 wherein R³ is a hydrogen atom or a C₁₋₆ alkyl group; Y is a single bond or a carbonyl group; Z is a methylene group or a carbonyl group; p3 is an integer of from 0 to 4; q3 is an integer of from 0 to 3; and p3 and q3 satisfies 0 ≤ p3 + q3 ≤ 7.

8. The radiation sensitive composition according to any one of claims 5 to 7, wherein the compound B is represented by the following formula 3: 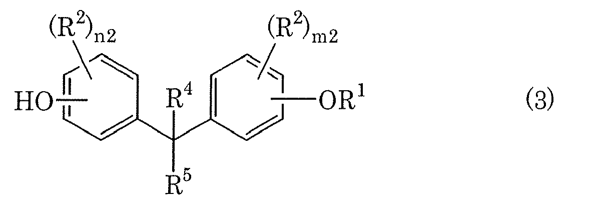 wherein R¹, R², R⁴, R⁵, m2 and n2 are the same as defined above, and two carbon atoms each being at o-position of respective two benzene rings with respect to -CR⁴R⁵- may be bonded to each other via an oxygen atom or a sulfur atom to from a xanthene structure or a thioxanthene structure represented by the following formula 4: 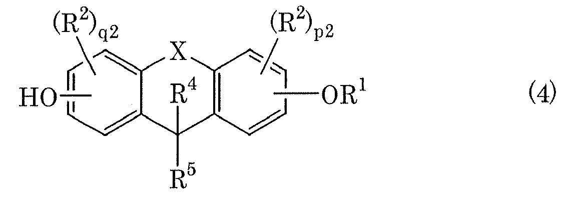 wherein R¹, R², R⁴, R⁵, X, p2 and q2 are the same as defined above.

9. The radiation sensitive composition according to any one of claims 1 to 8, wherein the compound B is produced by introducing the acid-dissociating functional group into 10 to 95% of the total number of the phenolic hydroxyl groups of the polyphenol compound A.

10. The radiation sensitive composition according to any one of claims 1 to 9, wherein the compound B dissolves in propylene glycol monomethyl ether acetate or ethyl lactate in an amount of 5% by weight or more at 23°C.

11. The radiation sensitive composition according to any one of claims 1 to 10, comprising from 5 to 40% by weight of the solid component and from 60 to 95% by weight of the solvent, and containing the compound B in an amount of from 80 to 99% by weight of a total weight of the solid component.

12. A compound represented by the following formula 1: 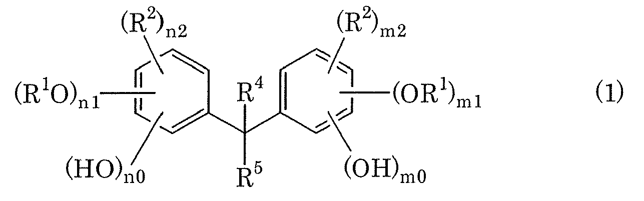 wherein each of R¹ groups is independently an acid-dissociating functional group selected from the group consisting of substituted methyl group, 1-substituted ethyl group, 1-substituted-n-propyl group, 1-branched alkyl group, silyl group, acyl group, 1-substituted alkoxymethyl group, cyclic ether group, and alkoxycarbonyl group;
each of R² group is independently a group selected from the group consisting of halogen atom, alkyl group, cycloalkyl group, aryl group, aralkyl group, alkoxy group, aryloxy group, alkyenyl group, acyl group, alkoxycarbonyl group, alkyloyloxy group, aryloyloxy group, cyano group, and nitro group;
R⁴ is hydrogen atom, C₁₋₆ alkyl group or aryl group, R⁵ is a monovalent C₁₀₋₁₈ group having a biphenyl structure or a naphthalene structure, or -CR⁴R⁵⁻may be a bivalent C₁₀₋₁₈ group having a fluorene structure, a acenaphthene structure, 1-ketoacenaphthene structure or a benzophenone structure when R⁴ and R⁵ are bonded to each other;
m0 and n0 are each an integer of from 0 to 3, m1 and n1 are each an integer of from 0 to 3, and m2 and n2 are each an integer of from 0 to 4, each satisfying the formulae: 1 ≤ m0 + m1 + m2 ≤ 5, 1 ≤ n0 + n1 + n2 ≤ 5, 1 ≤ m1 + n1 ≤ 6, 1 ≤ m0 + m1 ≤ 3, and 1 ≤ n0 + n1 ≤ 3; and
two carbon atoms of two benzene rings each being at ortho-position with respect to -CR⁴R⁵- may be bonded to each other via oxygen atom or sulfur atom to form a xanthene structure or a thioxanthene structure represented by the following formula 2: 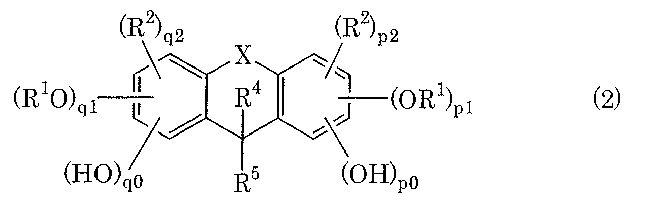 wherein R¹, R², R⁴ and R⁵ are the same as defined above; p0 and q0 are each an integer of from 0 to 2, p1 and q1 are each an integer of from 0 to 2, p2 and q2 are each an integer of from 0 to 3, each satisfying 1 ≤ p0 + p1+p2 ≤ 4, 1 ≤ q0 + q1 + q2 ≤ 4, 1 ≤ p1 + q1 ≤ 4, 1 ≤ p0 + p1 ≤ 2, and 1 ≤ q0 + q1 ≤ 2; and X is oxygen atom or sulfur atom.

13. The compound according to claim 12, wherein R⁵ is represented by the following formula: 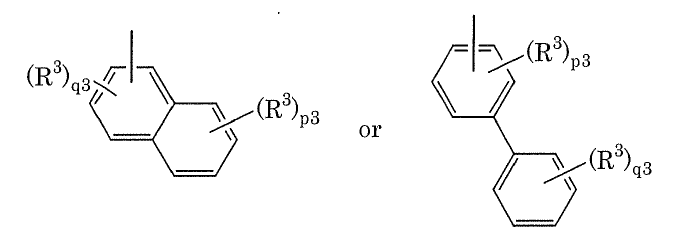 wherein R³ is a hydrogen atom or a C₁₋₆ alkyl group; p3 is an integer of from 0 to 4; q3 is an integer of from 0 to 3; and p3 and q3 satisfies 0 ≤ p3 + q3 ≤ 7.

14. The compound according to claim 12, wherein R⁴ and R⁵ of -CR⁴R⁵- are bonded to each other such that -CR⁴R⁵- forms a bivalent group represented by the following formula: 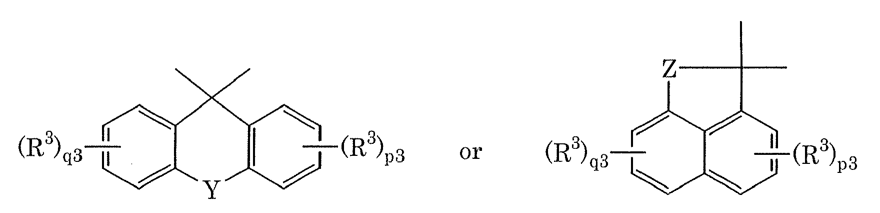 wherein R³ is a hydrogen atom or a C₁₋₆ alkyl group; Y is a single bond or a carbonyl group; Z is a methylene group or a carbonyl group; p3 is an integer of from 0 to 4; q3 is an integer of from 0 to 3; and p3 and q3 satisfies 0 ≤ p3 + q3 ≤ 7.

15. The compound according to any one of claims 12 to 14, represented by the following formula 3: 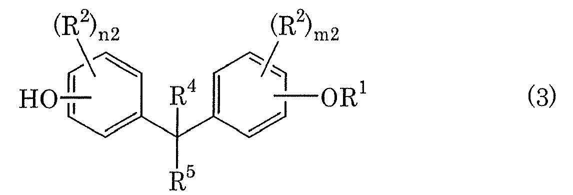 wherein R¹, R², R⁴, R⁵, m2 and n2 are the same as defined above, and two carbon atoms each being at o-position of respective two benzene rings with respect to -CR⁴R⁵- may be bonded to each other via an oxygen atom or a sulfur atom to from a xanthene structure or a thioxanthene structure represented by the following formula 4: 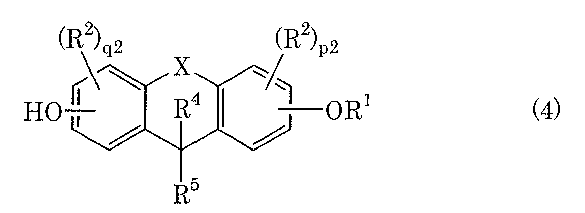 wherein R¹, R², R⁴, R⁵, X, p2 and q2 are the same as defined above.

16. The compound according to any one of claims 12 to 14, which is at least one compound selected from the group consisting of 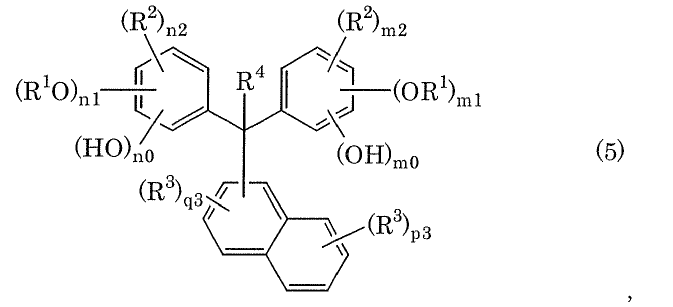 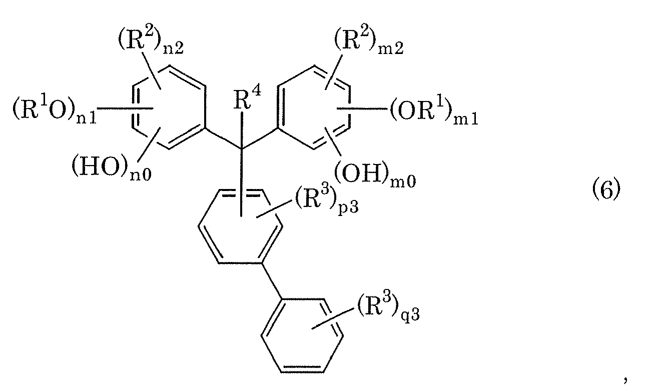 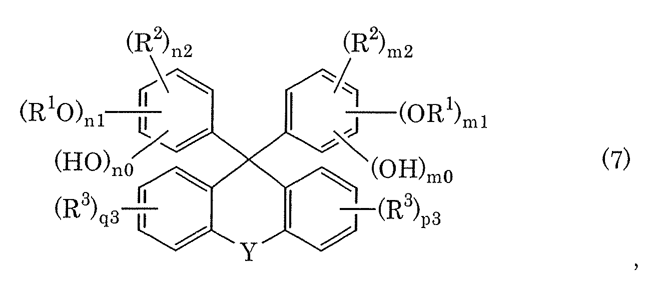 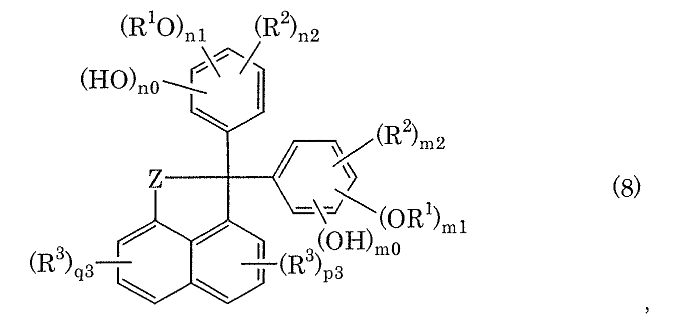 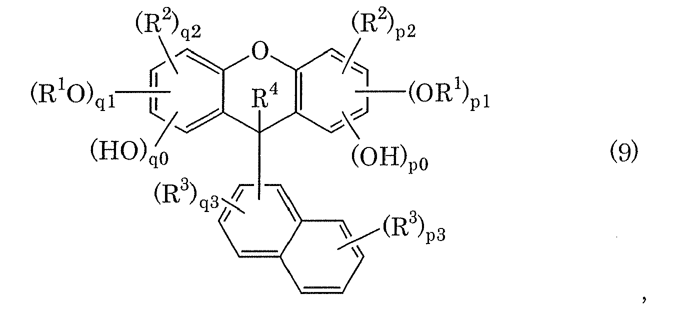 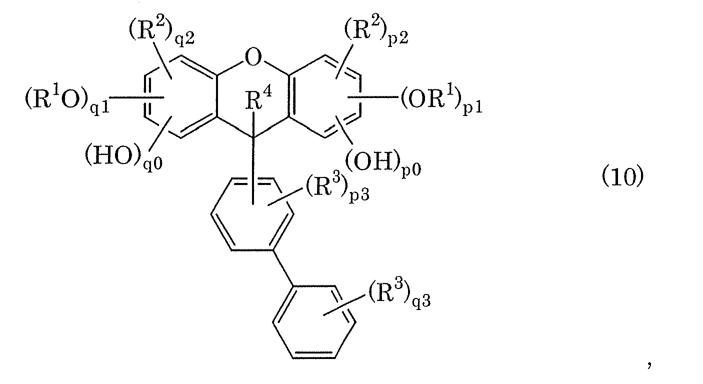 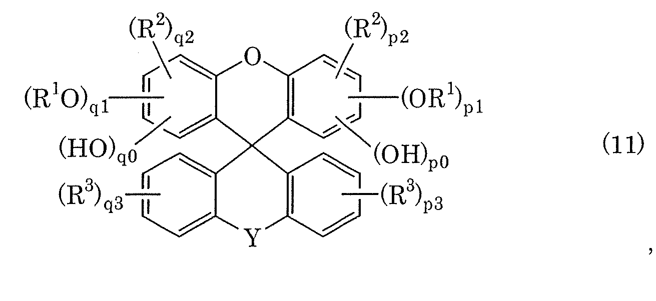 and 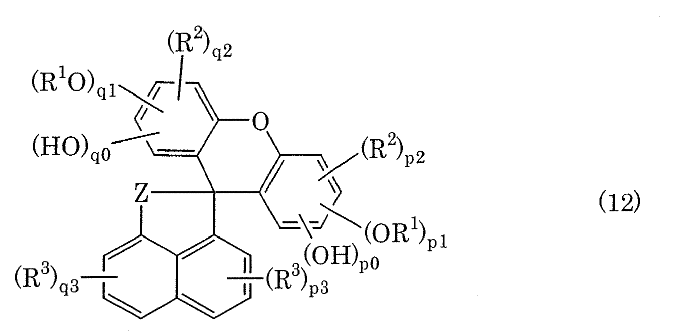 wherein R¹ to R⁴, Y, Z, m0 to m2, n0 to n2, p0 to p3, and q0 to q3 are as defined above.

17. The compound according to any one of claims 12 to 16, wherein a number of OR¹ group is from 10 to 95% of a total number of OR¹ group and OH group.

18. The compound according to any one of claims 12 to 17, which dissolves in propylene glycol monomethyl ether acetate or ethyl lactate in an amount of 5% by weight or more at 23°C.
